# EUROPÄISCHE PATENTSCHRIFT

(11) **EP 1 865 846 B1**
(45) Veröffentlichungstag und Bekanntmachung des Hinweises auf die Patenterteilung: **12.08.2015**
(21) Anmeldenummer: 06723174.6
(22) Anmeldetag: 02.03.2006
(51) Int. Cl.: A61B 5/151

(54) **STECHSYSTEM ZUR ENTNAHME EINER KÖRPERFLÜSSIGKEIT**
PIERCING SYSTEM FOR REMOVING A BODILY FLUID
SYSTEME DE PIQURE DESTINE AU PRELEVEMENT D'UN LIQUIDE ORGANIQUE

(30) Priorität: 03.03.2005 DE 102005009652; 15.12.2005 EP 05027429
(43) Veröffentlichungstag der Anmeldung: 19.12.2007
(73) Patentinhaber: Roche Diagnostics GmbH, 68305 Mannheim (DE); F. Hoffmann-La Roche AG, 4070 Basel (CH)
(72) Erfinder: DECK, Frank, 67150 Niederkirchen (DE); QUARDER, Ortrud, 69115 Heidelberg (DE); WEISS, Thomas, 68307 Mannheim (DE); HÖRAUF, Christian, 68723 Oftersheim (DE); KEIL, Michael, 67071 Ludwigshafen (DE); KONYA, Ahmet, 67165 Waldsee (DE); HARTTIG, Herbert, 67434 Neustadt (DE); BAADER, Felix, CH-3063 Ittingen (CH); LIST, Hans, 64754 Hesseneck-Kailbach (DE); EBERT, Karl-Peter, 64407 Fränkisch-Crumbach (DE)
(74) Vertreter: Pfeifer, Hans-Peter
(86) Internationale Anmeldenummer: PCT/EP2006/001922
(87) Internationale Veröffentlichungsnummer: WO 2006/092309

(56) Entgegenhaltungen:
- US-A- 4 895 147
- US-A1- 2003 083 685
- US-A1- 2003 199 892
- US-A1- 2004 127 818
- US-A1- 2004 260 325

## Beschreibung

Die Erfindung betrifft ein Stechsystem zur Entnahme einer Körperflüssigkeit aus der Haut eines Menschen oder Tieres. Die Körperflüssigkeit ist in der Regel Blut. In manchen Anwendungsfällen geht es aber auch um die Gewinnung einer Probe von interstitieller Flüssigkeit. Nachfolgend wird ohne Beschränkung der Allgemeinheit auf Blut als Beispiel, auch für andere aus der Haut gewinnbare Körperflüssigkeiten, Bezug genommen.

Das System besteht aus einer zur einmaligen Verwendung vorgesehenen (disposiblen) Stecheinheit mit einem Nadelelement zum Einstechen in die Haut und einem Stechgerät mit einem Antrieb für die Stechbewegung. Auch eine insbesondere zur Verwendung in einem solchen System geeignete Stecheinheit ist Gegenstand'der Erfindung.

Disposible Stecheinheiten werden seit langem benutzt, um für analytischdiagnostische Zwecke eine geringe Menge Blut aus einem Körperteil (meist aus einem Finger oder Ohrläppchen) zu entnehmen. In diesem Zusammenhang werden die Stecheinheiten üblicherweise als Lanzetten bezeichnet. Zum manuellen Einstechen vorgesehene Lanzetten werden beispielsweise in dem US-Patent 3,789,830 beschrieben. Sie werden in der Regel nur von medizinisch trainiertem Personal verwendet. Dennoch ist der Einstich mit erheblichem Schmerz verbunden.

Bereits seit langem werden Stechgeräte verwendet, die den Stechantrieb enthalten. Das Stechgerät kann disposibel mit einer fest integrierten Lanzette ausgebildet sein. In der Regel ist es jedoch mehrfach verwendbar und hat eine Halterung, mittels der jeweils eine Lanzette auswechselbar mit dem Stechantrieb gekoppelt werden kann. Da die Geräte und Lanzetten aneinander angepaßte, von dem gleichen Hersteller gelieferte Elemente sind, werden sie als "Stechsystem" oder "Blutentnahmesystem" bezeichnet.

Als Antriebselement für den in einem Gehäuse des Stechgerätes angeordneten Lanzettenantrieb dient meist eine Feder. Durch eine Lanzettenführung wird gewährleistet, daß die Stechbewegung auf einem vorbestimmten Einstichweg erfolgt. Zu Beginn der Entwicklung waren sehr einfache Konstruktionen des Antriebs gebräuchlich, bei denen die Lanzette unmittelbar an einem Ende einer in einem länglichen Gehäuse angeordneten Druckfeder befestigt war. Ein solches Stechsystem ist beispielsweise aus dem US-Patent 4,895,147 und 4,469, 110 bekannt. Eine andere frühe Konstruktion eines Stechgerätes ist in dem US-Patent 4,442,836 beschrieben. Dabei wird die Bewegung der Lanzette in Richtung auf die Hautoberfläche bis zu dem Umkehrpunkt (Vortriebsphase der Stechbewegung) von einer ersten Feder angetrieben, während als Antrieb für die Rückzugsbewegung der Lanzette (Rückzugsphase der Stechbewegung) eine zweite Feder dient, die ihre Wirkung entfaltet, nachdem die Kraftkopplung zwischen der ersten Feder und der Lanzette unterbrochen wurde.

Bei den üblichen Konstruktionen haben die Stechgeräte an ihrem in Einstichrichtung vorderen Ende eine Austrittsöffnung, aus der die Spitze der Lanzette für kurze Zeit austritt, um eine Wunde in einem Körperteil zu erzeugen, gegen das das vordere Ende des Stechgerätes gedrückt wird. Die Stechtiefe ist dabei durch den Abstand in Einstichrichtung zwischen der Position, die die Lanzettenspitze am Umkehrpunkt der Lanzettenbewegung erreicht und der Ebene einer Hautkontaktfläche definiert, die die Austrittsöffnung ringförmig umgibt und im Moment des Einstichs an der Haut anliegt. Das vordere Ende des Stechgerätes mit der Hautkon-taktfläche bildet also ein Stechtiefenreferenzelement, durch das gewährleistet wird, daß die Stechtiefe einem vorgegebenen Wert entspricht.

Um die Stechtiefe zu kontrollieren, ist es gebräuchlich, den Bewegungsweg der Lanzette in Einstichrichtung dadurch zu begrenzen, daß ein mit der Lanzette verbundenes Anschlagteil auf eine korrespondierende Anschlagfläche im Gehäuse des Stechgerätes auftrifft. Diese Gehäuseanschlag-Konstruktion ist beispielsweise in dem US-Patent 4,469,110 dargestellt. Bei dem in dem US-Patent 4,442,836 beschriebenen Antrieb mit zwei Federn soll eine definierte Position des Umkehrpunktes der Lanzettenbewegung dadurch gewährleistet werden, daß die Kraftübertragung zwischen der Antriebsfeder und der Lanzette an einem definierten Punkt des Bewegungsweges unterbrochen wird.

Derartige Blutentnahmesysteme wurden den hohen Anforderungen nicht gerecht, die zu erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Aktivität etc. stark schwankt) ihren Blutzuckerspiegel möglichst ständig innerhalb bestimmter Sollgrenzen zu halten. Durch umfangreiche wissenschaftliche Untersuchungen wurde bewiesen, daß mittels einer Intensivtherapie mit mindestens vier Blutänalysen pro Tag ein dramatischer Rückgang schwerster Spätschäden des Diabetes mellitus (beispielsweise einer Retinopathie mit resultierender Erblindung des Patienten) erreicht werden kann.

Diese Intensivtherapie setzt voraus, daß die Blutentnahme mit einem möglichst geringen Schmerz verbunden ist. Große Fortschritte in dieser Hinsicht wurden mit der in dem US-Patent 5,318,584 beschriebenen Konstruktion erreicht, die unter anderem auf der Erkenntnis basiert, daß der mit der Blutgewinnung verbundene Schmerz deutlich verringert werden kann, wenn das Stechsystem so ausgebildet ist, daß der Einstich (auch bei Verwendung von jeweils neuen disposiblen Lanzetten für aufeinanderfolgende Stechvorgänge) mit bis dahin nicht erreichter Qualität reproduzierbar ist. Um dies zu gewährleisten, wird ein Lanzettenantrieb mit einem Antriebsrotor verwendet, auf den einerseits (antriebsseitig) eine Antriebsfeder einwirkt und der andererseits über einem (abtriebsseitigen) Kopplungsmechanismus so mit der Lanzette gekoppelt ist, daß die Rotation des Antriebsrotors in die gewünschte Stechbewegung umgesetzt wird. Der abtriebsseitige Kopplungsmechanismus ist (mittels einer Steuerkurve) so gestaltet, daß die Lanzette während der gesamten (aus Vortriebsphase und Rückzugsphase bestehenden) Stechbewegung mit dem Antriebsrotor praktisch ohne Spiel gekoppelt ist und dadurch die Lanzettenbewegung vollständig durch die entsprechende Bewegung des Antriebsrotors kontrolliert wird. Durch diese Konstruktion werden einerseits die Erschütterungen vermieden, die bei dem Antriebstyp mit Gehäuseanschlag (US-Patent 4,469,110) mit dem Aufeinandertreffen der beiden Anschläge verbunden sind. Andererseits wird durch die permanente spielfreie Kopplung der Lanzette mit dem Antriebsrotor eine exakt reproduzierbare Position des Umkehrpunktes der Lanzettenbewegung bei wiederholten Stechbewegungen gewährleistet, die bei dem Antriebstyp des US-Patentes 4,442,836 nicht erreicht wird.

Die Erfindung bezieht sich nicht nur auf Stechsysteme, die ausschließlich dazu dienen, einen Blutstropfen für eine anschließende Analyse mit einem anderen Gerät zu gewinnen. Vielmehr richtet sie sich insbesondere auch auf sogenannte integrierte Systeme, mit denen nicht nur die Blutentnahme, sondern auch die Analyse durchgeführt wird, möglichst ohne zusätzliche Handhabungsschritte durch den Benutzer. Damit sind zusätzliche Anforderungen verbunden, die unter anderem aus der räumlichen Enge resultieren, wenn beide Funktionen in einem (aus Handhabungsgründen möglichst kleinen) Gerätegehäuse untergebracht werden müssen.

In den US-Patenten 8,029,583 und 5,514,152 werden integrierte Systeme beschrieben, bei denen die Blutgewinnung mit einer Lanzettennadel erfolgt, deren Bewegungsweg in Einstichrichtung, wie bei dem US-Patent 4,469,110, durch einen Gehäuseanschlag beschränkt ist. Die Übertragung des gewonnen Blutstropfens zu einem Analysesensor erfolgt im Falle des US-Patentes 5,514,152 mit Hilfe eines in dem Gerätegehäuse verlaufenden Kapillarkanals.

In dem US-Patent 5,938,679 wird ein Stechsystem beschrieben, dessen Stecheinheit wahlweise mit Kapillarröhrchen versehen ist, durch die Blut mit Hilfe von Kapillarkräften in das Innere des Gerätes gesaugt werden kann. Dies ist ein Beispiel für ein Stechsystem, dessen Nadelelement einen Kapillarkanal aufweist, durch den eine Körperflüssigkeit aus der Haut in das Innere der Stecheinheit transportiert werden kann. Ein weiteres Beispiel eines solchen Stechsystems ist in der US-Patentanmeldung US 2003/0018282 A1 beschrieben. Dabei umfaßt die Stecheinheit nicht nur die in die Haut einzustechende Nadel mit einem zum Transport der Probe geeigneten Kapillarkanal, sondern auch einen Reagenzien enthaltenden Nachweisbereich. Eine solche Stecheinheit, die gleichzeitig einen (beispielsweise als kapillaraktive saugschicht und/oder Hohlkammer ausgebildeten) Aufnahmebereich für die Probe hat und vorzugsweise auch die für die Analyse erforderlichen Reagenzien enthält, wird nachfolgend als "Mikrosampler" bezeichnet. Hinsichtlich näherer Einzelheiten über Mikrosampler wird auf die erwähnte US-Patentanmeldung und die darin zitierten Dokumente, insbesondere das US-Patent 5,801,057 verwiesen. Abgesehen von den hier beschriebenen Besonderheiten können im Rahmen der vorliegenden Erfindung Mikrosampler unterschiedlicher Konstruktionen eingesetzt werden.

Die vorliegende Erfindung richtet sich generell auf Stechsysteme unterschiedlicher Typen, wobei das Nadelelement, das in die Haut eingestochen wird, als massive Nadel (wie bei den erwähnten Lanzetten) oder als Kapillarnadel (mit offener Kapillare oder als geschlossene Hohlnadel) ausgebildet sein kann. Soweit auf Lanzetten oder andere speziellere Ausführungsformen Bezug genommen wird, geschieht dies beispielhaft und ohne Beschränkung der Allgemeinheit. Die erläuterten Sachverhalte gelten grundsätzlich auch für andere Ausgestaltungen.

Auf dieser Grundlage liegt der Erfindung die Aufgabe zugrunde, ein Stechsystem zu schaffen, bei dem eine reproduzierbare Stechtiefe in verbesserter Weise gewährleistet wird und die sich insbesondere zur Anwendung in integrierten Analysesystemen eignet.

Die Aufgabe wird gelöst durch ein Stechsystem mit den Merkmalen des Anspruchs 1.

Gegenstand der Erfindung ist auch eine disposible Stecheinheit gemäß Anspruch 21.

Im Gegensatz zu den gebräuchlichen Stechsystemen, bei denen das Stechtiefenreferenzelement von einem fest mit dem Gehäuse verbundenen Bauteil, normalerweise dem vorderen Ende des Gehäuses des Stechgerätes, gebildet wurde, ist für die Erfindung charakteristisch, dass das Stechtiefenreferenzelement mindestens während eines Teils der Vortriebsphase mit dem Nadelelement mitgeführt wird. Dieser Typ von Stechsystemen ist beispielsweise aus der US 2004/0127818 bekannt. Entscheidend für die Stechtiefe ist der "Überstand" der Nadelspitze gegenüber der Hautkontaktfläche, die an dem in Einstichrichtung vorderen Ende des mitgeführten Stechtiefenreferenzelementes vorgesehen ist. Dieser Überstand ist einstellbar, um die Einstellung unterschiedlicher Stechtiefen in Abhängigkeit von den Bedürfnissen des Benutzers zu ermöglichten.

Das Stechgerät kann ein zur einmaligen Verwendung vorgesehenes (disposibles) Produkt sein, wobei in diesem Fall die Stecheinheit zweckmäßigerweise untrennbar (über den Kopplungsmechanismus) mit dem Stechantrieb verbunden ist. Bevorzugt ist das Stechgerät jedoch mehrfach verwendbar und weist eine Halterung auf, mittels der jeweils eine disposible Stecheinheit auswechselbar mit dem Stechantrieb gekoppelt werden kann.

Wie nachfolgend noch näher erläutert wird, läßt sich die Einstellbarkeit des Nadelüberstandes gemäß der Erfindung dadurch erreichen, dass das Stechgerät getrennte Kupplungen für das Nadelelement einerseits und das Stechtiefenreferenzelement andererseits einschließt. Der Begriff "Kupplung" ist in diesem Zusammenhang allgemein als Verbindung zu verstehen, durch die Kräfte übertragen werden. Wie weiter unten noch näher erläutert wird, genügt es für manche Ausführungsformen der Erfindung, wenn die Kupplung in nur einer Bewegungsrichtung (unidirektional) wirkt. Bevorzugt wirkt jedoch mindestens die Nadelelement-Kupplung bidirektional. Jedenfalls schließt die Kupplung ein Positionierungsteil mit einem Anschlag ein, der derartig mit einem korrespondierenden Anschlag des Nadelelementes bzw. des Stechtiefenreferenzelementes zusammenwirkt, dass die Längsposition des Nadelelementes bzw. des Stechtiefenreferenzelementes mindestens an dem Umkehrpunkt der Stechbewegung durch den Kontakt der Anschlagflächen bestimmt wird. Der Nadelüberstand wird durch die Relativposition der beiden Positionierungsteile in Einstichrichtung bestimmt und ist somit durch Veränderung dieser Relativposition einstellbar.

Gemäß einer Ausführungsform wird der Nadelüberstand vor Beginn der Stechbewegung eingestellt und bleibt danach mindestens bis zum Erreichen des Umkehrpunktes, also während der Vortriebsphase der Stechbewegung, konstant. Dies hat zur Folge, dass das Stechtiefenreferenzelement während der Vortriebsphase mit gleicher Geschwindigkeit wie das Nadelelement, also synchron mit diesem, bewegt wird. Gemäß anderen Ausführungsformen wird die relative Längsposition der beiden Elemente der Stecheinheit noch während der Vortriebsphase verändert. Jedenfalls muß diese relative Längsposition zu dem Zeitpunkt, zu dem die Stechbewegung den Umkehrpunkt erreicht, einen definierten Wert haben, der dem vorbestimmten Wert der Stechtiefe entspricht.

Die Tatsache, dass erfindungsgemäß das Stechtiefenreferenzelement mit seiner Hautkontaktfläche bei der Stechbewegung mitgeführt wird, bedeutet nicht, dass bei erfindungsgemäßen Geräten keine gerätefeste Hautkontaktfläche vorhanden ist. Vielmehr ist gemäß einer bevorzugten Ausführungsform - insoweit übereinstimmend mit bekannten Systemen-am vorderen Ende des Stechgerätes eine zweite Hautkontaktfläche vorhanden, die eine Gehäuseöffnung ringförmig umgibt und mit der es bei der Benutzung gegen die Haut gedrückt wird. Im Gegensatz zu bekannten Geräten dient diese Gehäuse-Hautkontaktfläche jedoch nicht als Stechtiefenreferenz. Die Stechtiefenreferenz wird von der Referenzelement-Hautkontaktfläche gebildet.

Die von der Gehäuse-Hautkontaktfläche umschlossene Gehäuseöffnung hat vorzugsweise einen relativ großen Durchmesser von beispielsweise mindestens 3 mm, vorzugsweise mindestens 5 mm. Eine solche relativ große Öffnung ermöglicht die Realisierung zusätzlicher Funktionen, die insbesondere bei integrierten Systemen für die Aufnahme der für die Analyse erforderlichen Blutprobe vorteilhaft sind. Beim Andrücken des Gerätes wölbt sich die Haut aufgrund ihrer Elastizität in die relativ große Öffnung hinein. In welchem Ausmaß dieses Hineinwölben der Haut in die Gehäuseöffnung stattfindet, hängt von verschiedenen Faktoren, insbesondere dem Anpreßdruck und der Elastizität der Haut ab. Daraus resultiert eine Varianz der Position der Hautoberfläche, die hier als "Z-Varianz" bezeichnet wird. Auf der Grundlage der Erfindung kann trotz dieser Z-Varianz eine gute Reproduzierbarkeit der Stechtiefe erreicht werden.

Gemäß einer bevorzugten Ausführungsform ist das Stechgerät mit einem "Hubausgleich" ausgestattet, durch den der Umkehrpunkt der Stechbewegung zumindest teilweise an die jeweilige Position der Hautoberfläche angepaßt und dadurch die bei der Benutzung des Stechsystems auftretende Z-Varianz zumindest teilweise ausgeglichen wird. Als Hubausgleich wird demzufolge eine Einrichtung zur Anpassung der Stechbewegung bezeichnet, durch die eine zumindest näherungsweise Anpassung der Längsposition von deren Umkehrpunkt an die jeweilige Position der Hautoberfläche erreicht wird. Der Hubausgleich kann deshalb auch als Hubanpassungsmechanismus bezeichnet werden. Es sind unterschiedliche Ausgestaltungen möglich:
- Als "aktiv gesteuerter Hubausgleich" wird eine Konstruktion bezeichnet, bei der die jeweilige Position der Hautoberfläche (z.B. mit mechanischen, elektronischen oder optisch-elektronischen Mitteln) detektiert und die Stechbewegung (vor oder während der Vortriebsphase), durch Steuerungsmittel, die auf den Stechantrieb, dessen Positionierung in dem Stechgerät oder den Kopplungsmechanismus einwirken, an die jeweilige Position der Hautoberfläche angepaßt wird.
- Alternativ kann der Hubausgleich mittels eines elastischen Elementes realisiert sein, das eine "Pufferung" der Stechbewegung bewirkt. Dieses elastische Element kann Bestandteil des Stechantriebs oder des Kopplungsmechanismus zwischen Stechantrieb und Lanzette sein. Die Pufferung läßt sich aber auch durch elastische Lagerung des Stechantriebes erreichen. Als elastisches Element eignen sich insbesondere Metallfedern, jedoch können auch elastische Elemente verwendet werden, die aus elastomeren Materialien einschließlich Gummi bestehen.
- Der Hubausgleich kann auch mit Hilfe einer Friktionskupplung realisiert sein, in der zwei Kupplungselemente mittels Friktionskraft derartig verbunden sind, daß sie die Übertragung einer Kraft in Richtung der Stechbewegung ermöglichen, jedoch bei Überschreiten eines Grenzwertes der auf die Kupplung wirkenden Kraft gegeneinander derartig beweglich sind, daß die Kraftübertragung in Richtung der Stechbewegung unterbrochen wird.

Diese Konstruktionsprinzipien können auch in Kombination miteinander verwendet werden.

Ein wesentlicher Vorteil der Erfindung besteht darin, daß sie es ermöglicht, eine gut reproduzierbare Stechtiefe mit einer sehr raumsparenden und mechanisch relativ einfachen Konstruktion zu erreichen. Dadurch ist sie in besonderem Maße für integrierte Systeme geeignet, bei denen aus den oben erläuterten Gründen eine möglichst kompakte Bauweise angestrebt wird. Durch die relativ einfache Bauweise ist die Herstellung zu günstigen Kosten möglich.

Ein weiterer Vorteil resultiert daraus, daß die Reproduzierbarkeit der Stechtiefe weitgehend unabhängig von der Konstruktion des Stechantriebs ist. Die Erfindung kann in Verbindung mit ganz unterschiedlichen Stechantrieb-Varianten realisiert werden. Insbesondere können die Antriebsgeschwindigkeit und weitere Einzelheiten der Stechbewegung an die jeweiligen Erfordernisse angepaßt werden. Erforderlichenfalls kann der Antrieb so ausgebildet werden, daß die Stecheinheit nach dem Einstich rasch zurückgezogen wird und damit Raum gibt für nachfolgende Analysefunktionen, insbesondere die Aufnahme der Probe in ein Analyseelement.

Für mehrere Ausgestaltungen der Erfindung ist ein Stechantrieb bevorzugt, bei dem ein festes (nicht elastisches) Antriebselement aus einer Ausgangsposition in eine Endposition bewegt und diese Bewegung des Antriebselementes mittels des Kopplungsmechanismus in die Stechbewegung transformiert wird. Das Antriebselement ist vorzugsweise ein um eine zentrale Achse rotierender Rotor, jedoch sind auch andere Antriebselemente, beispielsweise Schwenk- oder Kniehebel, bekannt und - abhängig von den Anforderungen im Einzelfall- für die Erfindung geeignet. Die Bewegung des Antriebselementes erfolgt mit Federkraft oder mit anderen bekannten Mitteln, beispielsweise elektrisch oder elektromagnetisch.

In jedem Fall erzeugt der Stechantrieb eine translatorische Bewegung des Nadelelementes zwischen einer Ausgangsposition und dem Umkehrpunkt der Stechbewegung. Die Distanz zwischen diesen Punkten der Bewegung der Stecheinheit wird als Hub der Stechbewegung bezeichnet.

Die Erfindung wird nachfolgend anhand von in den Figuren dargestellten bevorzugten Ausführungsformen näher erläutert. Die darin dargestellten Besonderheiten können einzeln oder in Kombination verwendet werden, um bevorzugte Ausgestaltungen der Erfindung zu schaffen. Es zeigen:
- Fig. 1: eine schematische Schnittdarstellung der im Zusammenhang mit der Erfindung wesentlichen Komponenten einer ersten Ausführungsform eines Stechsystems;
- Fig. 2: eine Figur 1 entsprechende Darstellung in vier Benutzungspositionen a bis d des Stechsystems;
- Fig. 3: eine schematische Seitenansicht einer ersten Ausführungsform eines für die Erfindung geeigneten Stechantriebs mit Hubausgleich;
- Fig. 4: eine schematische Seitenansicht einer zweiten Ausführungsform eines für die Erfindung geeigneten Stechantriebs;
- Fig. 5: vier Benutzungspositionen eines Stechsystems mit einer weiteren Ausführungsform eines Hubausgleichs;
- Fig. 6: eine schematische Schnittansicht einer erfindungsgemäßen Stecheinheit;
- Fig. 7: eine schematische Schnittansicht einer weiteren Ausführungsform einer erfindungsgemäßen Stecheinheit;
- Fig. 8: eine Aufsicht auf die Lanzettenaufnahme einer Stecheinheit gemäß Figur 6;
- Fig. 9: eine Aufsicht auf die Unterseite des Lanzettenkörpers einer Lanzette der Stecheinheit gemäß Figur 6;
- Fig. 10: eine Seitenansicht einer weiteren Ausführungsform einer erfindungsgemäßen Stecheinheit in Form eines Mikrosamplers und des Kopplungsmechanismus eines zugehörigen Stechgerätes, mit dem der Mikrosampler ein Stechsystem bildet;
- Fig. 11: eine Vorderansicht der in Figur 10 dargestellten Komponenten;
- Fig. 12: eine Aufsicht von unten auf den in Figur 10 dargestellten Mikrosampler;
- Fig. 13: eine Aufsicht von oben auf den in Figur 10 dargestellten Mikrosampler;
- Fig. 14: eine Seitenansicht eines Mikrosamplers ähnlich Figur 11 in vier verschiedenen Benutzungspositionen a bis d;
- Fig. 15: ein Weg-Zeit-Diagramm einer für ein erfindungsgemäßes Stechsystem typischen Stechbewegung;
- Fig. 16: eine perspektivische Darstellung eines Teils eines bekannten Stechsystems mit magazinierten Lanzetten;
- Fig. 17: eine Schnittdarstellung eines Teils einer Ausführungsform der Erfindung mit magazinierten Stecheinheiten;
- Fig. 18: eine perspektivische Darstellung einer weiteren Ausführungsform eines erfindungsgemäßen Stechsystems ohne Gehäuse;
- Fig. 19: eine Schnittdarstellung des Stechsystems von Fig. 18;
- Fig. 20: ein Weg-Zeit-Diagramm einer weiteren für ein erfindungsgemäßes Stechsystem typischen Stechbewegung;
- Fig. 21: eine schematische Seitenansicht eines Stechsystems ähnlich Figur 18 in vier Benutzungspositionen A bis D;
- Fig. 22: eine Seitenansicht einer weiteren Ausführungsform eines erfindungsgemäßen Stechsystems ohne Gehäuse;
- Fig. 23: eine Längsschnitt-Darstellung des Stechsystems von Fig. 22;
- Fig. 24: eine perspektivische Darstellung des Stechsystems von Fig. 22;
- Fig. 25: eine Seitenansicht (teilweise im Schnitt) einer weiteren Ausführungsform eines erfindungsgemäßen Stechsystems;
- Fig. 26: einen schematischen, nicht maßstäblichen Querschnitt durch ein Nadelelementband
- Fig. 27 bis 30: eine weitere Ausführungsform eines erfindungsgemäßen Stechsystems in vier Benutzungspositionen, jeweils in perspektivischer Ansicht (A) und in Seitenansicht, teilweise geschnitten (B).
- Fig. 31 bis 33: eine Teil-Querschnittsdarstellung einer weiteren Ausführungsform eines erfindungsgemäßen Stechsystems in drei Benutzungspositionen

Soweit die Worte "vorne" und "hinten" zur Bezeichnung der Lokalisierung von Bauteilen verwendet werden, bezieht sich dies auf die Einstichrichtung. Beispielsweise wird also das Ende der Stecheinheit oder des Stechgerätes, mit dem bzw. an dem der Einstich erfolgt, als vorderes Ende und das gegenüberliegende Ende als hinteres Ende bezeichnet. Die Bezeichnungen "Längsrichtung" und "Längsposition" beziehen sich auf die Raumrichtung der Stechbewegung, die auch als Z-Richtung bezeichnet wird und bei den üblichen länglichen ("Pencil-förmigen") Stechgeräten mit deren Hauptachse übereinstimmt.

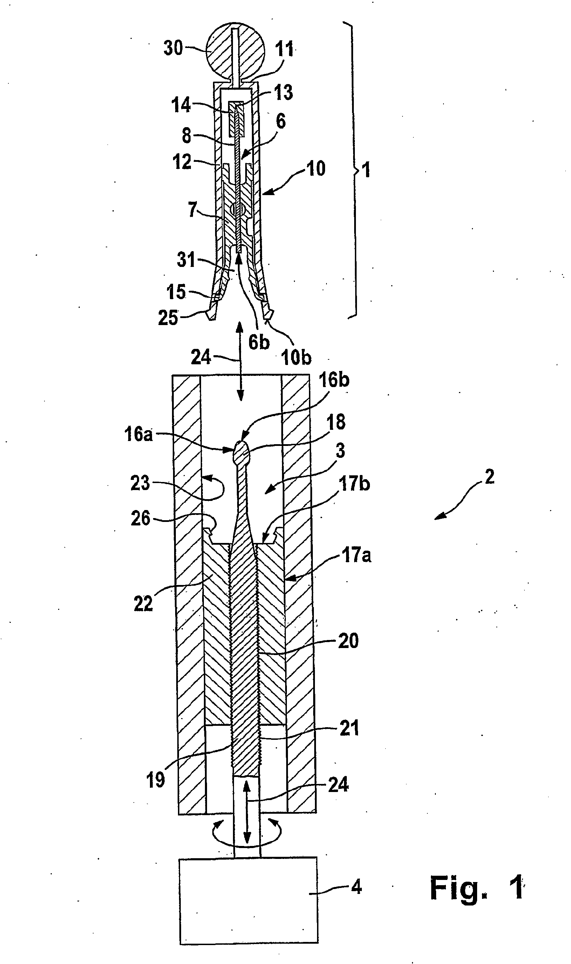

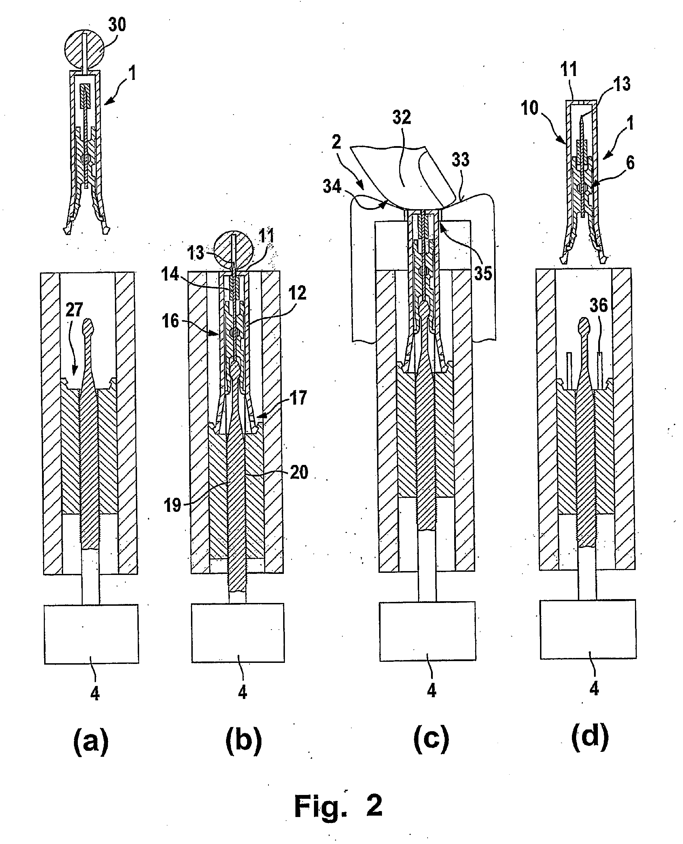

Die Figuren 1 und 2 zeigen die für die erfindungsgemäße Funktion wesentlichen Komponenten eines Stechsystems, nämlich eine disposible Stecheinheit 1 und Teile eines in seiner Gesamtheit nicht dargestellten Stechgerätes 2. Diese Teile bilden vor allem den Kopplungsmechanismus 3 zur Verbindung einer Stecheinheit 1 mit einem in diesen Figuren nur symbolisch dargestellten Stechantrieb 4.

Die Stecheinheit 1 hat zwei Hauptbestandteile, nämlich ein insgesamt mit 6 bezeichnetes Nadelelement mit einem Nadelelementkörper 7 und einer Nadel 8 sowie ein Stechtiefenreferenzelement 10 mit einer Hautkontaktfläche 11 und einem Referenzelementkörper 12, der sich von der Hautkontaktfläche 11 nach hinten erstreckt und den Körperteil 7 des Nadelelementes 6 umgreift. Wenn sich die Nadel 8 in der in den Figuren 1 und 2a dargestellten Benutzungsposition befindet, ist ihre Spitze 13 von einem Sterilschutz 14 umgeben, der vorzugsweise aus einem die Nadelspitze 13 eng umschließenden und dadurch dessen Sterilität während der Lagerung der Stecheinheit 1 gewährleistenden Kunststoffmaterial besteht.

In den Figuren 1 und 2a ist der Lieferzustand der Stecheinheit 1 dargestellt. In diesem Zustand ist das Nadelelement 6 durch Fixierungsmittel 15 in einer definierten Längsposition innerhalb des Referenzelementkörpers 12 fixiert, wobei die Fixierungsmittel 15 (beispielsweise in Form der dargestellten, in passende Ausnehmungen eingreifenden Noppen) so ausgebildet sind, daß die Fixierung bei dem nachfolgend erläuterten Einsetzen der Stecheinheit (oder spätestens während der Stechbewegung) gelöst wird, wenn eine Relativverschiebung des Nadelelementes 6 gegenüber dem Stechtiefenreferenzelement 10 notwendig ist.

Der Kopplungsmechanismus 3 weist getrennte Kupplungen für die Elemente der Stecheinheit 1 auf, nämlich eine Nadelelementkupplung 16 und eine Referenzelementkupplung 17. Jede der Kupplungen hat ein Positionierungsteil 16a bzw. 17a mit einem Anschlag 16b bzw. 17b, der mit einem korrespondierenden Anschlag 6b des Nadelelementes 6 bzw. 10b des Stechtiefenreferenzelementes 10 derartig zusammenwirkt, daß im gekoppelten Zustand der Elemente deren jeweilige Längsposition mindestens an dem Umkehrpunkt der Stechbewegung durch den Kontakt dieser Anschläge bestimmt wird.

Im dargestellten Fall wird das Positionierungsteil 16a der Nadelelementkupplung 16 von dem verdickten Kopf 18 einer Verbindungsstange 19 gebildet, über die das Nadelelementpositionierungsteil 16a mit dem Stechantrieb 4 verbunden ist. Die vordere Begrenzungsfläche des Kopfes 18 bildet den Anschlag 16b. Der korrespondierende Anschlag 16b des Nadelelementes 6 wird bei der dargestellten Konstruktion von dem hinteren Ende der Nadel 8 gebildet. Die dargestellte Konstruktion der Nadelelementkupplung ist aus der US 2004/0260325 bekannt, aus der weitere Informationen entnommen werden können.

Als Positionierungsteil 17a der Referenzelementkupplung 17 dient ein Gleitkörper 22, der in einer zylindrischen Bohrung 23 sitzt. Er hat eine Axialbohrung mit einem Innengewinde 20, in das die mit einem entsprechenden Außengewinde 21 versehene Verbindungsstange 19 eingeschraubt ist. Durch gegenseitiges Drehen der von diesen Bauelementen gebildeten Positionierungsteile 16a,17a kann deren Längsposition relativ zueinander verändert werden. Vorzugsweise ist das (hier von dem Gleitkörper 22 gebildete) Referenzelement-Positionierungsteil 17a drehfest und axial verschieblich gelagert, während das (hier von dem Kopf 18 der Positionierungsstange 19 gebildete) Nadelelement-Positionierungsteil 16a mit einem Bauteil (hier der Verbindungsstange 19) verbunden ist, dessen Position durch Drehen um die eigene Achse innerhalb des Referenzelement-Positionierungsteils 17a in Längsrichtung veränderbar ist.

Bei der in Figur 1 durch einen Doppelpfeil 24 symbolisierten aus Vortriebsphase und Rückzugsphase bestehenden Stechbewegung wirken der Gleitkörper 22 und die Bohrung 23 als Führung, durch die gewährleistet wird, daß die Stechbewegung präzise entsprechend der vorgegebenen Einstichrichtung erfolgt.

Die Referenzelementkupplung ist im dargestellten Fall als Rasthaken-Konstruktion ausgebildet. An dem in radialer Richtung elastisch nachgiebigen hinteren Ende des Referenzelementkörpers 12 sind Rasthaken 25 ausgebildet, die beim Einsetzen der Stecheinheit 1 in ein entsprechendes, an der vorderen Stirnseite des Gleitkörpers 22 ausgebildetes Rastprofil 26 eingreifen. Dabei werden die Anschläge 17b bzw. 10b von einer Stirnfläche des Gleitkörpers 22 bzw. dem hintersten Ende des Referenzelementkörpers 12 gebildet.

Die Nadelelementkupplung 16 und die Referenzelementkupplung 17 bilden insgesamt eine Halterung 27, mittels der jeweils eine disposible Stecheinheit 1 auswechselbar mit dem Stechantrieb 4 eines mehrfach verwendbaren Stechgerätes 2 gekoppelt werden kann. Das Einsetzen einer Stecheinheit 1 in die Halterung 27 ist in den Teilfiguren a und b von Figur 2 dargestellt.

Zur leichteren Handhabung weist die Stecheinheit 1 eine beispielsweise im Kunststoffspritzverfahren angespritzte Einsetzhilfe 30 auf, die nach dem Einsetzen von dem Benutzer abgedreht wird. Beim Einsetzen wird die Stecheinheit 1 von der in Figur 2a dargestellten Ausgangsposition in die in Figur 2b dargestellte Position gebracht, bei der sowohl die Nadelelementkupplung 16 als auch die Referenzelementkupplung 17 geschlossen sind und eine in beide axiale Richtungen (bidirektional) wirkende feste Verbindung zwischen der Verbindungsstange 19 und dem Nadelelementkörper 7 bzw. zwischen dem Gleitkörper 22 und dem Referenzelementkörper 12 bilden. Dabei umschließt eine am hinteren Ende des Nadelelementkörpers 7 vorgesehene Aufnahme 31 den Kopf 18 der Verbindungsstange 19. Die Rasthaken 25 verhaken mit dem entsprechenden Rastprofil 26. Aus dem Vergleich der Teilfiguren a und b ist zu erkennen, daß durch die Relativbewegung des Nadelelementes 6 gegenüber dem Stechtiefenreferenzelement 10 der Sterilschutz 14 von der Nadelspitze 13 weg nach hinten geschoben wurde.

Der Abstand in Längsrichtung des Anschlags 16b gegenüber dem Anschlag 17b ist vorzugsweise schon vor dem Einsetzen der Stecheinheit 1 so eingestellt, daß das Nadelelement 6 während des Einsetzvorganges (unter Lösen der Fixierungsmittel 15) nach vorne geschoben wird bis die Nadelspitze 13 aus der Hautkontaktfläche 11 heraustritt und der Überstand der gewünschten Stechtiefe entspricht. Es sind jedoch auch Ausführungsformen möglich, bei denen die Stechtiefe erst nach dem Einsetzen der Stecheinheit eingestellt wird. In jedem Fall kann die exakte gewünschte Stechtiefe durch Verändern der relativen Längsposition der Positionierungsteile 16a,17a eingestellt werden. Wenn die Einstellung vor der Stechbewegung erfolgt, bleibt der Überstand der Nadelspitze 13 gegenüber der Hautkontaktfläche 11 während der Stechbewegung konstant und die Position der Stecheinheit wird zu jedem Zeitpunkt der Stechbewegung von der jeweiligen Position der Verbindungsstange 19 bestimmt.

Figur 2c zeigt die Systemkomponenten im Moment des Einstichs in eine Fingerkuppe 32. Dabei liegt die Haut auf einer nach innen (zur Hauptachse des Gerätes) und hinten konisch geneigten Gehäuse-Hautkontaktfläche 33 auf, welche die Einstichstelle umgibt. Sie ist an dem in dieser Teilfigur teilweise dargestellten vorderen Ende des Stechgerätes 2 ausgebildet ist. Durch diese Gehäuse-Hautkontaktfläche 33 wird eine hinreichend definierte Längsposition der Hautoberfläche 34 in der Umgebung der Einstichstelle relativ zu dem Stechantrieb 4 gewährleistet. Die Stechtiefe wird durch den Abstand der Nadelspitze 13 von der Hautkontaktfläche 11 bestimmt. Da die von der gerätefesten Hautkontaktfläche 33 umrahmte Gehäuseöffnung 35 relativ groß ist, wölbt sich allerdings die Hautoberfläche 34 in die Gehäuseöffnung 35 hinein, wobei das Ausmaß dieser Vorwölbung von verschiedenen Faktoren, insbesondere dem Anpreßdruck und der Elastizität der Haut abhängt. Dies führt zu der oben erwähnten Z-Varianz der Einstichstelle der Hautoberfläche 34.

Figur 2d zeigt die Systemkomponenten beim Auswerfen der Stecheinheit. Zu diesem Zweck ist ein Auswerfer, beispielsweise in Form der hier dargestellten Stangen 36, vorgesehen, der zum Auswerfen der Stecheinheit 1 mittels eines nicht dargestellten Antriebs nach vorne bewegt wird. Die Konstruktion ist so gestaltet, daß das Stechtiefenreferenzelement 10 zunächst nach vorne geschoben wird, während das Nadelelement 6 noch fixiert ist. Durch die resultierende Relativverschiebung der Elemente 6,10 tritt die Nadelspitze 13 hinter die Hautkontaktfläche 11 zurück bis sie sich geschützt im Innern des Stechtiefenreferenzelementes 10 befindet. Dadurch wird eine Verletzungsgefahr durch die scharfe Nadelspitze 13 und das damit verbundene Infektionsrisiko vermieden.

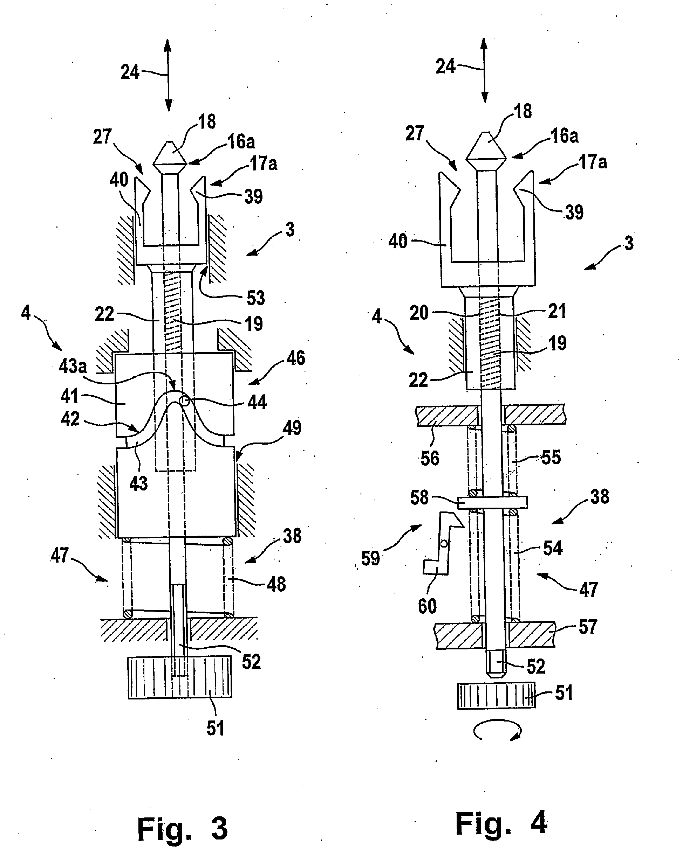

Die Figuren 3 und 4 zeigen zwei Ausführungsformen eines Stechantriebs 4, der einen allgemein mit 38 bezeichneten Hubausgleich aufweist, um die Stechbewegung an die jeweilige Position der Hautoberfläche (innerhalb der bei unterschiedlichen Stechvorgängen möglichen Z-Varianz) anzugleichen. Übereinstimmend mit den Figuren 1 und 2 weist bei beiden Ausführungsformen der Kopplungsmechanismus 3 eine Verbindungsstange 19 mit einem Außengewinde 21 auf, das in einer entsprechenden Gewindebohrung 20 eines Gleitkörpers 22 sitzt. Auch in diesem Fall bildet der Kopf 18 der Gewindestange 19 ein Positionierungsteil 16a einer Nadelelementkupplung, während ein Positionierungsteil 17a einer Referenzelementkupplung an dem Gleitkörper 22 ausgebildet ist.

Auch das mechanische Konstruktionsprinzip der Nadelelementkupplung und der Referenzelementkupplung stimmt mit den Figuren 1 und 2 überein. Die Positionierungsteile 16a, 17a haben jeweils ein Kupplungsprofil, das mit einem entsprechenden Kupplungsprofil in einem Kupplungsbereich des nicht dargestellten Nadel- bzw. Referenzelementes zusammenwirkt, wobei eine (vorzugsweise bidirektional wirkende) Kupplung mit entsprechenden Anschlagelementen realisiert ist.

Abweichend von den Figuren 1 und 2 wird bei den Figuren 3 und 4 das Positionierungsteil 17a von nach innen (auf die Achse der Verbindungsstange 19 zu) gerichteten Vorsprüngen 39 elastischer Arme 40 gebildet. An den entsprechenden Nadelelementen sind nach außen offene Vertiefungen oder Ausnehmungen vorgesehen, in die die Vorsprünge 39 eingreifen.

Die Positionierungsteile 16a und 17a bilden eine Halterung 27 zur auswechselbaren Aufnahme von jeweils einer Stecheinheit. Durch gegenseitiges Verdrehen der Verbindungsstange 19 und des Gleitkörpers 22 kann - wiederum übereinstimmend mit den Figuren 1 und 2 - die Längsposition der Positionierungsteile 16a und 17a relativ zueinander verändert und dadurch der Nadelüberstand einer angekoppelten Stecheinheit eingestellt werden. Die Position der Halterung 27 und somit einer angekoppelten Stecheinheit wird zu jedem Zeitpunkt der Stechbewegung von der jeweiligen Position der Verbindungsstange 19 bestimmt.

Als Antriebselement des in Figur 3 dargestellten Stechantriebs 4 dient ein Antriebsrotor 41, der (angetrieben von einer nicht dargestellten Antriebsfeder und nach Lösen eines ebenfalls nicht dargestellten Auslösers) eine Rotationsbewegung um seine Längsachse ausführt, die mit der Achse der Verbindungsstange 19 übereinstimmt. Die Rotation des Antriebsrotors 41 wird durch eine Kurvensteuerung 42, welche eine Steuerkurve 43 und einen Steuerkurvenreiter 44 umfaßt, in die translatorische Stechbewegung entsprechend dem Doppelpfeil 24 umgesetzt. Derartige Rotorantriebe sind aus verschiedenen Veröffentlichungen (z.B. dem oben zitierten US-Patent 5,318,584) bekannt, so daß eine Erläuterung konstruktiver Einzelheiten nicht erforderlich ist.

Der Antriebsrotor 41 und die Kurvensteuerung 42 bilden ein Drehschiebegetriebe 46, durch das die Rotation des Antriebsrotors 41 in die Stechbewegung umgewandelt wird, wobei der Umkehrpunkt der Stechbewegung erreicht wird, wenn der Steuerkurvenreiter 44 den Scheitelpunkt 43a der Steuerkurve 43 durchläuft. Das Drehschiebegetriebe 46 gewährleistet eine exakte und eindeutige Zuordnung zwischen der jeweiligen Position des Antriebselementes (Rotors) und der Stecheinheit. Ein Stechantrieb, der diese Bedingung erfüllt, wird als "zwangsgeführt" bezeichnet.

Bei der dargestellten Ausführungsform wird ein solcher zwangsgeführter Stechantrieb mit einem Hubausgleich durch ein elastisches Bauelement 47 kombiniert. Im dargestellten Fall ist dieses elastische Bauelement eine Schraubenfeder 48, die so ausgebildet und angeordnet ist, daß sie ein elastisches Lager für den Antriebsrotor 41 bildet. Der Rotor 41 ist in einer Führungsbohrung 49 derartig axial verschieblich gelagert, daß er gegen die Kraft der Schraubenfeder 48 nach hinten bewegt werden kann, wenn die Hautkontaktfläche des Stechtiefenreferenzelementes einer mit der Halterung 27 verbundenen Stecheinheit bei der Stechbewegung auf die Hautoberfläche auftrifft. Dieser Zeitpunkt wird als "Kontaktierungszeitpunkt" bezeichnet.

Durch den Hubausgleich, hier durch die Elastizität des elastischen Bauelementes 47, wird die Position des Umkehrpunktes an die Z-Position der Hautoberfläche angepaßt. Die elastische Federkraft muß so bemessen sein, daß das elastische Bauelement während der Stechbewegung vor dem Kontaktierungszeitpunkt nicht wesentlich deformiert wird. Jedenfalls sollte es unmittelbar vor dem Kontaktierungszeitpunkt praktisch nicht deformiert sein. Die Pufferwirkung soll erst bei Kontakt der Hautkontaktfläche mit der Haut eintreten.

Zur Einstellung der Stechtiefe dient ein Einstellrad 51. Die DrehmomentÜbertragung von dem in axialer Richtung unbeweglichen Einstellrad auf die axial bewegliche Verbindungsstange 19 kann mit Hilfe einer Längsverzahnung 52 der Verbindungsstange erfolgen, die in eine entsprechende Innenverzahnung des Einstellrades 51 eingreift.

Zur Führung des Referenzelement-Positionierungsteils 17a (somit auch einer in der Halterung 27 befestigten Stecheinheit) dient eine Gehäusebohrung 53, die im dargestellten Fall im Bereich der elastischen Arme 40 positioniert ist. Die erforderliche elastische Beweglichkeit der Arme 40 wird durch nicht dargestellte längs verlaufende Nuten gewährleistet, wobei durch eine nicht-runde Querschnittsgestaltung der Bohrung 53 eine axial bewegliche aber drehfeste Führung gewährleistet wird.

Auch bei der in Figur 4 dargestellten Ausführungsform des Stechantriebs 4 wird ein Hubausgleich mittels eines elastischen Bauelementes 47 erreicht. Es besteht in diesem Fall aus zwei Federn, nämlich einer Antriebsfeder 54 und einer Rückhubfeder 55, die jeweils einerseits an einem gehäusefesten Lagerteil 56 bzw. 57 befestigt sind und andererseits über einen Verbindungsflansch 58 auf die Verbindungsstange 19 einwirken.

In Figur 4 ist der Ruhezustand der Federn 54 und 55 dargestellt. Um den Antrieb zu spannen, wird (mit nicht dargestellten Mitteln) die Verbindungsstange 19 nach hinten bewegt, bis ein Triggerelement 59, das beispielsweise aus einer mit dem Verbindungsflansch 58 zusammenwirkenden Klinke 60 bestehen kann, einrastet. Die Verschiebung der Verbindungsstange 19 beim Spannen führt dazu, daß eine auch bei dieser Ausführungsform am Ende der Verbindungsstange 19 vorgesehene Längsverzahnung 52 in eine entsprechende Innenverzahnung eines Einstellrades 51 eingreift, so daß durch Drehen des Einstellrades 51 der Überstand der Nadelspitze einer mit der Halterung 27 verbundenen Stecheinheit eingestellt werden kann.

Nach dem Lösen des Triggerelements 59 wird die Verbindungsstange 19 von der Antriebsfeder 54 nach vorne getrieben. Durch die elastische Gestaltung des Antriebs endet die Vortriebsphase der Stechbewegung unmittelbar nach dem Kontaktierungszeitpunkt. Die Längsdimension der Bauteile ist so gewählt, daß im gesamten Varianzbereich der Position der Hautoberfläche die Antriebsfeder 54 über ihre Ruheposition hinaus gedehnt bzw. die Rückhubfeder 55 über ihre Ruheposition hinaus komprimiert ist, wenn die Hautkontaktfläche einer in der Halterung 27 fixierten Stecheinheit auf die Hautoberfläche auftrifft. Demzufolge wird unmittelbar nach dem Kontaktierungszeitpunkt der Umkehrpunkt der Stechbewegung erreicht und anschließend die Rückzugsphase durch die in diesem Zustand überwiegende Kraft der Rückhubfeder 55 angetrieben.

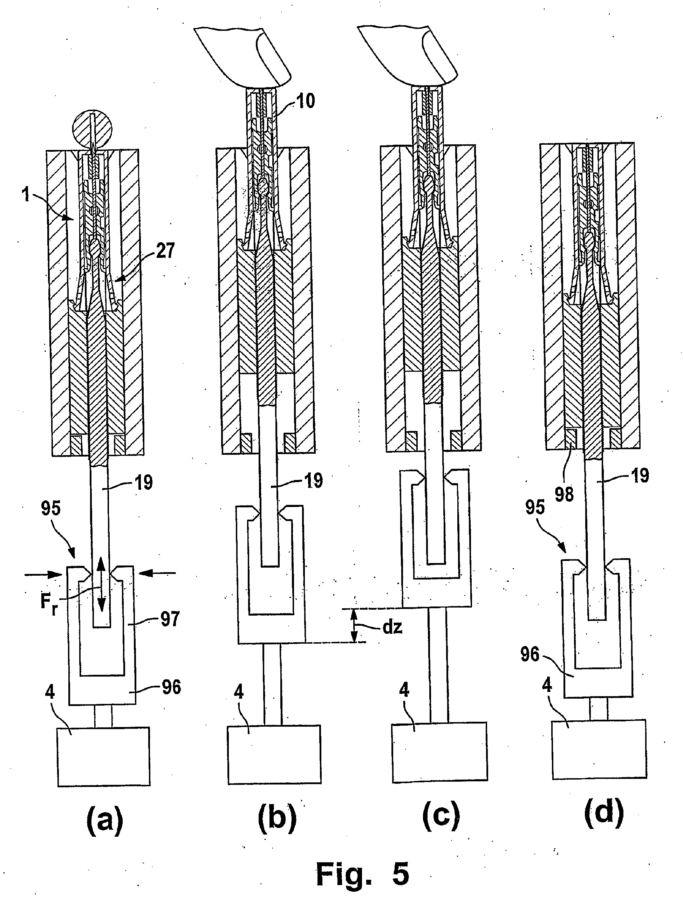

Figur 5 zeigt vier Funktionspositionen (a) bis (d) eines Stechsystems mit einem Hubausgleich, der mittels einer Friktionskupplung 95 realisiert ist. Sie besteht im dargestellten Fall aus der Verbindungsstange 19 und einer Zange 96, deren Arme 97 gegen die Verbindungsstange 19 drücken. Die Verbindungsstange 19 bildet auch bei dieser Ausführungsform die Verbindung zu einer Halterung 27 für eine Stecheinheit 1. Deren Konstruktion einschließlich der Nadelelementkupplung, der Referenzelementkupplung und der Einstellung des Nadelüberstandes durch Drehen der Verbindungsstange 19 stimmt mit den Figuren 1 und 2 überein.

Die Zange 96 und die Verbindungsstange 19 bilden zwei Kupplungselemente der Friktionskupplung 95, die die Übertragung einer Kraft in Richtung der Stechbewegung ermöglichen. Diese Kraft wird als "Friktionskraft Fᵣ" bezeichnet und ist durch die Reibungsverhältnisse zwischen den Elementen der Friktionskupplung, hier also der Zange 96 und der Verbindungsstange 19 bestimmt. Wenn die zwischen den Kupplungselementen 19,96 wirkende Kraft die Friktionskraft Fᵣ überschreitet, werden sie derartig gegeneinander beweglich, daß die Kraftübertragung in Richtung der Stechbewegung unterbrochen wird. Dies wird durch die Teilfiguren (a) bis (c) verdeutlicht:
- Während der Vortriebsphase der Stechbewegung (d.h. zwischen den in den Teilfiguren (a) und (b) dargestellten Funktionspositionen) ist die Friktionskraft Fᵣ größer als die in dieser Bewegungsphase auftretenden Kräfte, so daß sich die Relativposition der Elemente 19,95 nicht ändert.
- Teilfigur (b) zeigt den Kontaktierungszeitpunkt. Durch das Auftreffen der Hautkontaktfläche des Stechtiefenreferenzelementes 10 auf die Haut steigt die zum weiteren Vorwärtsbewegen des Nadelelementes erforderliche Kraft deutlich an und überschreitet den durch die voreingestellte Friktionskraft Fᵣ vorgegebenen Grenzwert. Dadurch findet eine Relativbewegung zwischen den Elementen der Friktionskupplung statt, die den erforderlichen Hubausgleich ermöglicht. Im dargestellten Fall wird der Hub um einen Betrag dz verkürzt, der der Differenz der Position der Zange 96 zwischen dem Hautkontaktzeitpunkt (Position (b)) und der maximalen Auslenkung des Lanzettenantriebs 4 (Position (c)) entspricht.
- Während der Rückzugsphase der Stechbewegung bleibt die Relativposition der beiden Kupplungselemente zunächst unverändert, bis die weitere Rückwärtsbewegung der Stecheinheit-Halterung durch eine geeignete Bewegungsbegrenzung 98 gestoppt wird. Während der weiteren Bewegung des Antriebs 4 erfolgt eine Relativbewegung der Elemente 96,19 der Friktionskupplung 95, bis sie sich wieder in der Ausgangsposition befinden.

Für diese Funktion ist erforderlich, daß die Friktionskraft Fᵣ einen definierten Wert hat. Sie muß so dimensioniert werden, daß sie größer als die Summe derjenigen Kräfte ist, die während der Stechbewegung vor dem Kontaktierungszeitpunkt auftreten. Dies sind im wesentlichen die dynamischen Beschleunigungskräfte zum Bewegen der beteiligten Massen und die statischen Stechkräfte zum Eindringen in die Haut. Andererseits muß die Friktionskraft Fᵣ kleiner sein als die maximal gewünschte Kraft, mit der das Stechtiefenreferenzelement 10 gegen die Haut gedrückt werden soll. Derartige Bedingungen können mit üblichen Materialien und Herstellungsverfahren eingestellt werden.

Bei den in den Figuren 3 bis 5 dargestellten Varianten des Hubausgleichs erfolgt die Anpassung an die jeweilige Z-Position der Hautoberfläche allein durch die Elastizität eines Bauelementes beziehungsweise durch die Friktionskupplung ohne gezielte Steuerung. Falls damit kein ausreichend komfortabler Einstich erreicht wird, kann ein aktiv gesteuerter Hubausgleich verwendet werden, wobei die aktive Steuerung auf einer Detektion der Position der Hautoberfläche basiert, welche beispielsweise mechanisch oder elektronisch erfolgen kann. Die elektronische Variante umfaßt selbstverständlich auch optisch-elektronische Detektionsverfahren.

Hinsichtlich der Steuerung des Umkehrpunktes der Stechbewegung kann man dabei folgende Grundprinzipien unterscheiden:
a) Die Adaption des Umkehrpunktes der Lanzettenbewegung kann vor Beginn der Stechbewegung erfolgen, insbesondere indem man den Stechantrieb in Längsrichtung verschiebt und dadurch den Abstand des Stechantriebs von der Hautoberfläche so einstellt, daß der Umkehrpunkt der Stechbewegung mit der jeweiligen Position der Hautoberfläche näherungsweise übereinstimmt. Idealerweise liegt der Umkehrpunkt der Stechbewegung etwas weiter vorne als die Position der Hautoberfläche zum Zeitpunkt des Hautkontaktes, wobei durch diese kleine "Hubreserve" die elastische Deformation der Hautoberfläche beim Einstich berücksichtigt wird.
   Bei dieser Ausführungsform kann vorteilhaft ein zwangsgeführter Lanzettenantrieb verwendet werden, wie er beispielhaft anhand des Rotorantriebs von Figur 3 erläutert wurde. Die Distanz zwischen Ausgangsposition und Umkehrpunkt der Stechbewegung, also der Betrag des Hubs, bleibt dabei unverändert. Der Hubausgleich erfolgt durch Längsverschiebung der Hubbewegung.
   Dieses Konstruktionsprinzip ermöglicht einen besonders präzisen Hubausgleich, wobei die Längsposition des Umkehrpunktes unabhängig von den Eigenschaften der Haut, insbesondere deren Elastizität, ist.
b) Die Detektion der Hautoberfläche kann während der Stechbewegung bei oder kurz vor dem Kontaktierungszeitpunkt erfolgen, wobei die Beschleunigung in Richtung auf die Haut unterbrochen und der Rückhub mit möglichst geringer zeitlicher Verzögerung nach der Detektion eingeleitet wird.
   Eine solche Variante kann konstruktiv relativ einfach mittels eines Tastelementes am vorderen Ende der Stecheinheit realisiert werden. Das Tastelement wirkt mit einem, vorzugsweise ebenfalls mechanischen, Steuerelement (beispielsweise einer Klinke) zusammen, das die Vortriebsphase beendet und die Rückzugsphase der Stechbewegung einleitet. Selbstverständlich sind auch in diesem Zusammenhang elektronische Varianten, insbesondere mit einem elektronischen Sensor am vorderen Ende der Stecheinheit, möglich.
   Bei diesem Konstruktionsprinzip hängt im Gegensatz zu a) der Hub der Stechbewegung von der Position der Hautoberfläche ab. Je näher sich die Hautoberfläche bei dem Stechantrieb befindet, desto früher wird die Vortriebsphase beendet und die Rückzugsphase eingeleitet, d.h. desto kleiner ist der Hub.

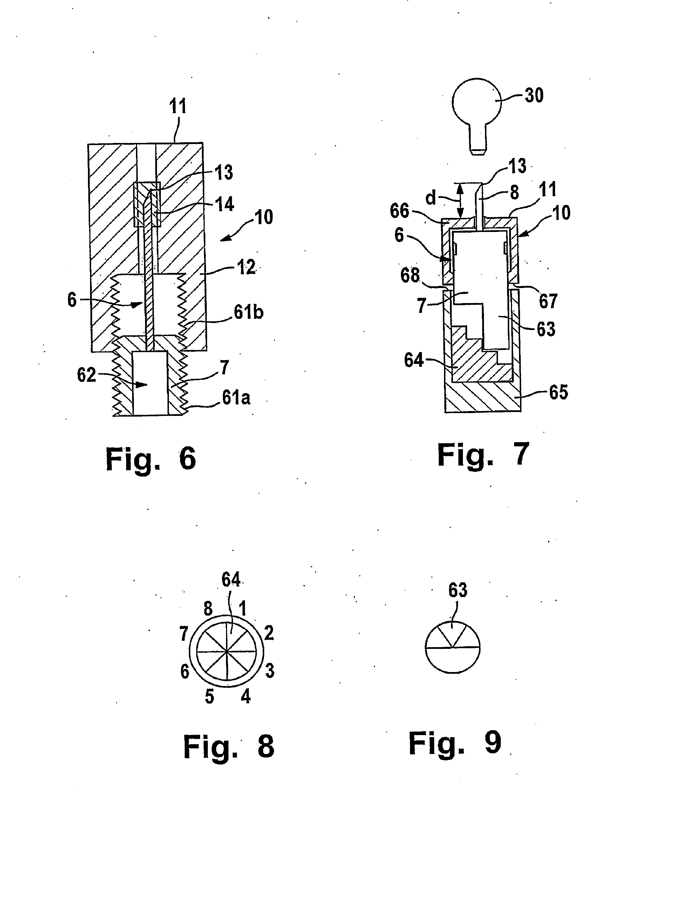

Die Figuren 6 und 7 bis 9 zeigen zwei Varianten erfindungsgemäßer Stecheinheiten, die für die manuelle Benutzung geeignet sind, aber auch als Bestandteil eines Stechsystems verwendet werden können. Dabei ist die Längsposition des Stechtiefenreferenzelementes 10 relativ zu dem Nadelelement 6 in der Einstichrichtung (welche der Orientierung der Nadel entspricht) auch ohne Stechgerät veränderbar und es sind Mittel vorhanden, durch die das Nadelelement 6 in einer eingestellten Position fixiert wird.

Bei der in Figur 6 dargestellten Ausführungsform weisen der Körperteil 7 des Nadelelementes 6 und der Referenzelementkörper 12 korrespondierende Schraubgewinde auf, die so ausgebildet und angeordnet sind, daß ihre relative Längsposition, somit auch die relative Längsposition der Lanzettenspitze 13 relativ zu der Hautkontaktfläche 11, durch gegenseitiges Verdrehen veränderbar ist. Um die Nadelspitze 13 aus der därgestellten Ruheposition innerhalb eines Sterilschutzes 14 in eine Benutzungsposition zu bringen, bei der sie gegenüber der Hautkontaktfläche 11 hervorsteht, wird der Körperteil 7 des Nadelelementes 6 mittels eines Werkzeugs gedreht, das in ein geeignetes Greifprofil 62 des Körperteils 7 eingreift. Dies kann manuell geschehen. Bevorzugt wird die in Figur 6 dargestellte Stecheinheit in Verbindung mit einem Stechgerät verwendet, wobei die Einstellung der Stechtiefe auch in diesem Fall manuell vor dem Einsetzen in das Stechgerät oder innerhalb des Stechgerätes erfolgen kann.

Bei der in Figur 7 dargestellten Variante ist an dem hinteren Ende des Körperteils 7 des Nadelelementes 6 ein Stützprofil 63 vorgesehen, das mit einem entsprechenden mehrstufigen Stützprofil 64 einer Stecheinheitaufnahme 65 zusammenwirkt. Eine mögliche Gestaltung der Stützprofile im Querschnitt, nämlich als Kreissektoren, ist in den Figuren 8 und 9 zu erkennen.

Das Stechtiefenreferenzelement 10 wird in diesem Fall von einer Kappe 66 gebildet, die den vorderen Teil des Körperteils 7 des Nadelelementes umgreift. Die Kappe 66 ist auf dem Körperteil 7 reibschlüssig fixiert. Im Ausgangszustand sitzt die in Figur 7 getrennt eingezeichnete Einsetzhilfe 30 über der Nadel 8. Sie schützt die Nadelspitze 13 und dient dazu, die Stecheinheit 1 in einer Drehposition in die Stecheinheitaufnahme 65 einzusetzen, die der gewünschten Stechtiefe entspricht. Beim Einsetzen wird die Kappe 66 relativ zu der Nadel 8 nach vorne geschoben, wenn ihre Unterkante 67 die Oberkante 68 der Stecheinheitaufnahme 65 kontaktiert. Dadurch wird der Überstand d der Nadelspitze 13 gegenüber der Hautkontaktfläche 11 eingestellt. Nach dem Einsetzen wird die Einsetzhilfe 30 abgedreht, so daß die Nadel 8 zur Benutzung frei liegt.

Es sind zahlreiche weitere Varianten erfindungsgemäßer Stecheinheiten möglich, die auch ohne Stechgerät verwendet werden können. Dabei ist es generell vorteilhaft, wenn .sich der Referenzelementkörper von der Hautkontaktfläche nach hinten erstreckt und einen Körperteil des Nadelelementes zumindest so weit umgreift, daß die Längsposition des Nadelelementes relativ zu dem Stechtiefenreferenzelement durch Kontakt zwischen dem Referenzelementkörper und dem Körperteil des Nadelelementes fixiert ist. Der Kontakt sollte mindestens reibschlüssig (wie bei Figur 7) sein. Eine formschlüssige Fixierung des Nadelelementes innerhalb des Referenzelementkörpers (wie bei Figur 6) ist für manche Anwendungszwecke besonders vorteilhaft.

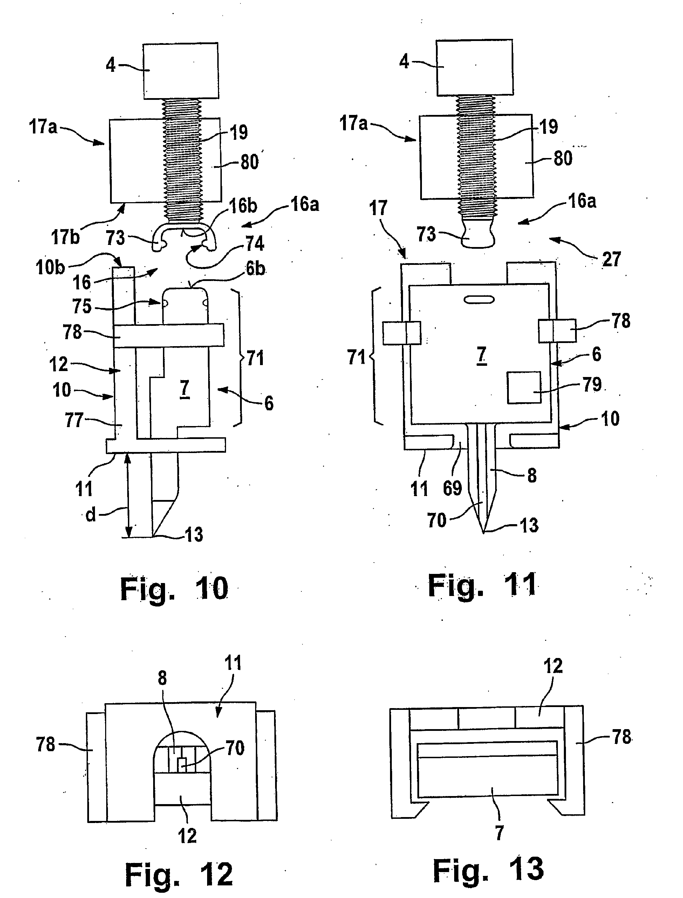

Die Figuren 10 bis 13 zeigen eine Stecheinheit, die sich von den zuvor dargestellten Stecheinheiten zunächst dadurch unterscheidet, daß die Nadel 8 des Nadelelementes 6 einen Kapillarkanal 70 aufweist, durch dessen Lumen Körperflüssigkeit in einen Probenaufnahmebereich 71 des Nadelelementes 6 strömen kann. Das Nadelelement 6 ist somit ein Mikrosampler der eingangs erläuterten Art. In dem Probenaufnahmebereich 71 sind (im Inneren des Körperteils 7 des Nadelelementes 6) Analysemittel angeordnet.

Auch bei dieser Ausführungsform basiert die Einstellung der Stechtiefe darauf, daß der Abstand d der Nadelspitze 13 von der Hautkontaktfläche 11 eines Stechtiefenreferenzelementes 10 mit Hilfe von Positionierungsteilen (16a,17a) eingestellt wird, die Bestandteile einer Nadelelementkupplung (16) und einer Referenzelementkupplung (17) sind.

Das Nadelelementpositionierungsteil 16a ist in diesem Fall als zweiarmige Greifklammer 73 ausgebildet, deren nach innen gewandte Arme elastisch sind und Vorsprünge 74 aufweisen, die in entsprechende Ausnehmungen 75 des Körperteils 7 des Nadelelementes 6 eingreifen, wenn die Nadelelementkupplung 16 geschlossen wird. Im geschlossenen Zustand der Nadelelementkupplung 16 wird die Längsposition des Nadelelementpositionierungsteils 16a durch das Zusammenwirken von zwei Anschlägen 16b,6b bestimmt, die im dargestellten Fall einerseits durch eine innere Begrenzungsfläche der Greifklammer 73 und andererseits durch die hintere Stirnseite des Nadelelementkörpers 7 gebildet werden. Auch diese Nadelelementkupplung wirkt bidirektional, koppelt also das Positionierungsteil 16a mit dem Nadelelement 6 in beiden Bewegungsrichtungen der Stechbewegung.

Der Referenzelementkörper 12 des Stechtiefenreferenzelementes 10 besteht im wesentlichen aus einem offenen Rahmen 77, an dessen Unterseite die Hautkontaktfläche 11 ausgebildet ist und quer zu der Längsrichtung verlaufenden das Nadelelement 6 umgreifenden Profilteilen 78. Die Hautkontaktfläche umgibt eine Öffnung 69 für die Nadel 8 des Nadelelementes 6.

Dieses Beispiel zeigt, daß der Referenzelementkörper kein geschlossenes Bauteil sein muß. Vielmehr ist auch eine offene Struktur geeignet, sofern sie die im Rahmen der Erfindung erforderlichen Funktionen erfüllt. Hierzu gehört insbesondere, daß das Nadelelement 6 mit dem Referenzelement 10 derartig verbunden ist, daß eine Relativbewegung beider Bauteile in Längsrichtung zur Einstellung des Nadelüberstandes d möglich ist. Außerdem sollte die Verbindung zwischen dem Nadelelement 6 und dem Referenzelement 7 eine gute Führung in Längsrichtung bewirken, so daß Bewegungen beider Bauteile in von der Einstichrichtung verschiedenen Raumrichtungen verhindert werden.

Das Positionierungsteil 17a der Referenzelementkupplung 17 mit einem Anschlag 17b ist an einem axial geführten aber rotationsfesten Lagerteil 80 ausgebildet. Ein korrespondierender Anschlag 10b wird von der hinteren Stirnseite des Rahmens 77 gebildet. In diesem Fall handelt es sich um eine unidirektional wirkende Kupplung, d.h. das Zusammenwirken der Anschläge 17b,10b bestimmt die relative Längsposition des Stechtiefenreferenzelementes 10 nur während der Vortriebsphase der Einstichbewegung (in den Figuren 10 und 11 nach unten), nicht während der Rückzugsphase.

Ähnlich wie bei den Figuren 1 bis 4 ist der Lanzettenantrieb 4 über eine Verbindungsstange 19 mit der von den Kupplungen 16,17 gebildeten Stecheinheit-Halterung 27 verbunden, wobei die relative Längsposition der Positionierungsteile 16,17 dadurch einstellbar ist, daß die Verbindungsstange 19 (an der das Referenzelementpositionierungsteil 16a befestigt ist) durch Drehen um ihre eigene Achse innerhalb des Referenzelementpositionierungsteils 17a (hier des Lagerteils 80) verschiebbar ist.

Die Greifklammer 73 ist am Ende der Verbindungsstange 19 drehbar gelagert und in dem Stechgerät so geführt, das sie unabhängig von der Drehposition der Verbindungsstange 19 in der in Figur 10 dargestellten Greifposition bleibt. Im Bereich der Vorsprünge 74 der Greifklammer 73 und der korrespondierenden Ausnehmungen 75 können elektrische Kontakte vorgesehen sein, mittels der elektrische Messungen an einer in dem Probenaufnahmebereich befindlichen Probenflüssigkeit durchgeführt werden können, wenn die Stecheinheit 6 als elektrochemischer Mikrosampler ausgebildet ist. Alternativ kann innerhalb des Probenaüfnahmebereiches 71 ein Fenster 79 vorgesehen sein, um im Falle eines mit photometrischer Analyse arbeitenden Mikrosamplers die erforderliche photometrische Messung zu ermöglichen. Da diese Meßprinzipien bekannt sind, ist eine nähere Erläuterung nicht erforderlich.

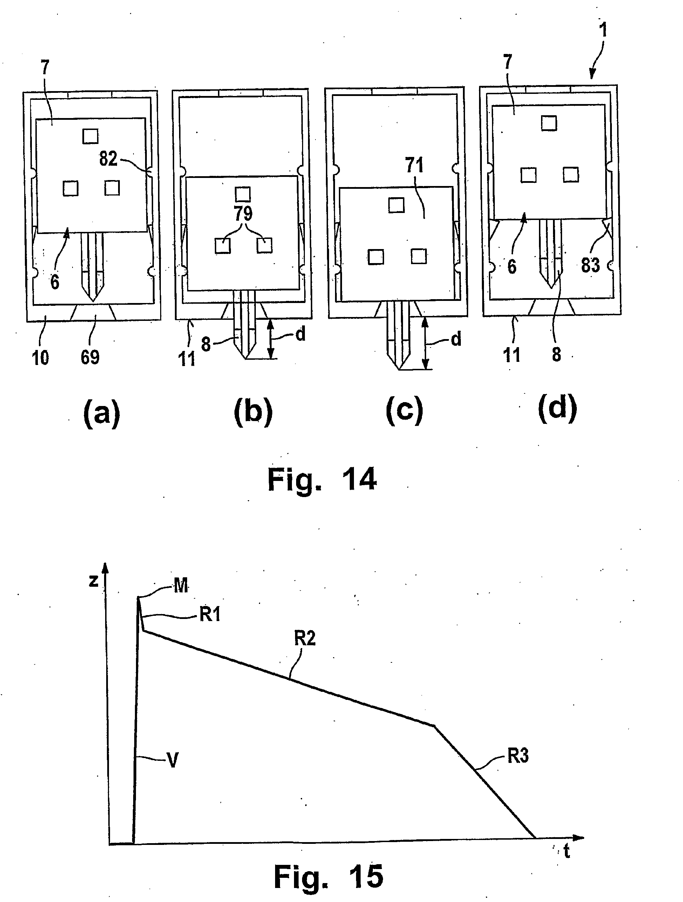

Figur 14 zeigt vier Benutzungsphasen (a) bis (d) einer Mikrosampler-Stecheinheit, deren konstruktive Merkmale den Figuren 10 bis 13 entsprechen:
- Teilfigur (a) zeigt den Lieferzustand, bei dem das Nadelelement 6 reibschlüssig in einer bestimmten Längsposition relativ zu dem Referenzelement 10 fixiert ist. Diese Fixierung wird durch Noppen 82 an dem Referenzelement 10 erreicht, die gegen Begrenzungsflächen des Körperteils 7 des Nadelelements 6 drücken.
- Teilfigur (b) zeigt den Zustand am Umkehrpunkt der Stechbewegung für den Fall eines Einstichs mit einem verhältnismäßig geringen Nadelüberstand d, also einer relativ geringen Einstechtiefe. Bei der Bewegung von dem Zustand (a) in den Zustand (b) wurde zunächst die Nadelelementkupplung 16 (Figur 10 und 11) geschlossen und das Nadelelement 6 nach unten gedrückt, wobei die Anschläge 16b,6b aneinander anliegen. Zu einem späteren Zeitpunkt während der Vortriebsphase erfolgte der Kontakt zwischen den Anschlägen 10b,17b, so daß auch das Referenzelement 10 in Richtung auf die Haut bewegt wird. Dieser Bewegungsablauf ist ein Beispiel dafür, daß die Einstellung der Stechtiefe nicht vor Beginn der Stechbewegung erfolgen muß, sondern auch während deren Vortriebsphase erfolgen kann. Die Nadel 8 dringt in die Haut ein und die Vortriebsphase der Stechbewegung wird beendet, wenn die Hautkontaktfläche 11 an der Haut anliegt.
- Die Teilfigur (c) zeigt ebenfalls den Benutzungszustand am Umkehrpunkt der Stechbewegung, jedoch bei maximalem Nadelüberstand d, also maximaler Stechtiefe.
- Wenn eine ausreichende Probenmenge in den Probenaufnahmebereich 71 des Nadelelementes 6 geströmt ist, wird die Rückzugsphase der Stechbewegung eingeleitet und das Nadelelement innerhalb des Referenzelementes zurückgezogen. Dabei wird das Referenzelement mit in den Figuren nicht dargestellten Mitteln festgehalten. Das Nadelelement 6 gelangt in die in Teilfigur (d) dargestellte Position, bei der die Nadel 8 zum Schutz gegen ein Verletzungsrisiko hinter die Hautkontaktfläche 11 zurückgezogen ist. In dieser Position sitzt der Körperteil 7 des Nadelelementes 6 hinter Sperrvorsprüngen 83, die so ausgebildet sind, daß das Nadelelement 6 nicht mehr zurück in die Benutzungsposition (Figuren b bzw. c) bewegt werden kann. Durch derartige Sperrmittel, die an dem Referenzelementkörper 12 und/oder dem Nadelelement 6 vorgesehen sein können, wird nach der Benutzung der Stecheinheit 1 die Bewegung des Nadelelementes 6 relativ zu dem Referenzelement 10 derartig beschränkt, daß eine nochmalige Benutzung einer benutzten Stecheinheit 1 zuverlässig verhindert wird.

Der Ablauf der Stechbewegung unterscheidet sich bei einem Mikrosampler (Figuren 10 bis 14) von der typischen Stechbewegung einer Lanzette (Figuren 1 bis 9). Während im Falle einer Lanzette die Rückzugsphase der Stechbewegung unmittelbar nach Erreichen des Umkehrpunktes stattfindet und die Stechbewegung auch insgesamt möglichst schnell ablaufen soll, wird im Falle eines Mikrosamplers die Bewegung der Stecheinheit während der Rückzugsphase nach Erreichen des Umkehrpunktes für die zum Ansaugen der Körperflüssigkeit erforderliche Zeitspanne unterbrochen oder verlangsamt. Ein entsprechendes Weg-Zeit-Diagramm ist in Figur 15 dargestellt. Man erkennt einen steilen Anstieg, der einer raschen Bewegung in Einstichrichtung während der Vortriebsphase V entspricht. Nach Erreichen eines Maximums M, das dem Umkehrpunkt der Stechbewegung entspricht, folgt bei der dargestellten bevorzugten Ausführungsform zunächst eine kurze und schnelle Rückzugsbewegung R1, die dann in eine langsame Rückzugsbewegung R2 übergeht. Der mit R1 bezeichnete Abschnitt der Rückzugsphase dient dazu, innerhalb des Hautgewebes im Bereich vor der Nadelspitze einen kleinen Freiraum zu bilden, in dem sich Probenflüssigkeit sammelt. Während des anschließenden Abschnitts R2 strömt das Blut durch den Kapillarkanal des Mikrosamplers in dessen Probenaufnahmebereich. Wenn dieser Vorgang abgeschlossen ist, folgt der Abschnitt R3 der Rückzugsphase, in dem die Nadel aus dem Hautgewebe herausgezogen wird.

Ein derartiger für ein Mikrosampler geeigneter Bewegungsablauf kann auf unterschiedliche Art und Weise realisiert werden. Insbesondere eignet sich für die Vortriebsphase V und den ersten Abschnitt der Rückzugsphase R1 ein mechanischer Federabtrieb, während die relativ langsame und kontrollierte Bewegung während der Abschnitte R2 und R3 vorteilhaft mit einem Elektromotor angetrieben wird.

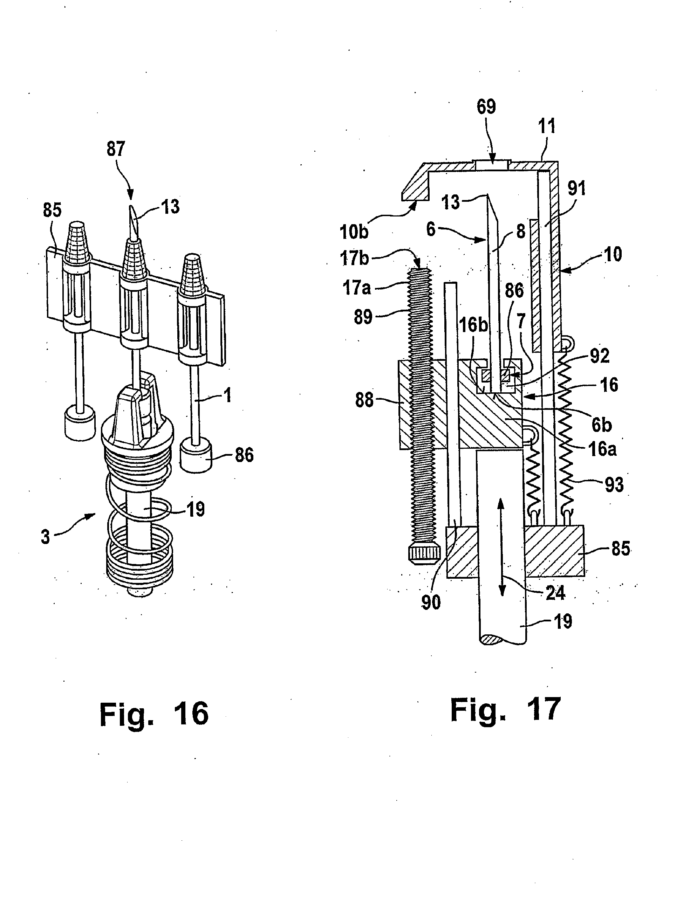

Die Figuren 16 und 17 sollen verdeutlichen, daß die Erfindung auch bei Stechsystemen verwendet werden kann, bei denen eine Mehrzahl von Stecheinheiten in einem Magazin zusammengefaßt sind und innerhalb des Stechgerätes nacheinander in eine Stechposition transportiert werden. Eine hierfür geeignete aus dem US-Patent 6,616,616 bekannte Konstruktion ist in Figur 16 dargestellt. Dabei befinden sich lanzettenförmige Stecheinheiten 1 in einem Magazinstreifen 85 aus Kunststoff, in dem sie so gehalten werden, daß sie axial beweglich und geführt sind. An ihrem hinteren Ende haben sie einen Kupplungszylinder 86. Er dient dazu, eine Lanzette, die sich in einer Stechposition 87 befindet, mit einem Kopplungsmechanismus 3 zu kuppeln, der die Verbindung zu einem nicht dargestellten Stechantrieb bildet.

Figur 17 verdeutlicht Abwandlungen, die geeignet sind, um die Erfindung bei einem Stechsystem mit magazinierten Stecheinheiten zu realisieren. Die Übertragung der von einem nicht dargestellten Stechantrieb erzeugten Stechbewegung erfolgt mittels einer Verbindungsstange 19, die relativ zu dem Magazinstreifen 85 die Stechbewegung 24 ausführt. Der Kopplungszylinder 86 bildet bei dieser Ausführungsform den Körperteil 7 des Nadelelementes 6. Die Verbindungsstange 19 wirkt auf ein Lagerteil 88, in das eine Stellschraube 89 eingeschraubt ist. Das Lagerteil 88 und ein Stechtiefenreferenzelement 10 mit einer Hautkontaktfläche 11 sind mittels Führungsstangen 90,91 in Richtung der Stechbewegung geführt.

Bei dieser Konstruktion wird das Positionierungsteil 16a der Referenzelementkupplung 16 von dem Lagerteil 88 gebildet. Die hintere Bodenfläche einer in dem Lagerteil 88 vorhandenen den Kopplungszylinder aufnehmenden Ausnehmung 92 bildet einen Anschlag 16b, der mit einem korrespondierenden Anschlag 6b zusammenwirkt, welcher in diesem Fall von dem hinteren Ende der Nadel 8 des Nadelelementes 6 gebildet wird.

Die Referenzelementkupplung 17 wirkt bei dieser Ausführungsform unidirektional. Ihr Positionierungsteil 17a wird von der Stellschraube 89 gebildet, deren vordere Stirnfläche als Anschlag 17b der Referenzelementkupplung wirkt, der mit einem korrespondierenden Anschlag 10b des Referenzelementes 10 zusammenwirkt.

Wenn in der Vortriebsphase der Stechbewegung die Verbindungsstange 19 nach vorne (in Figur 17 nach oben) geschoben wird, wird zunächst das Nadelelement 6 in Einstichrichtung bewegt, bis die Nadelspitze 13 aus der von der Hautkontaktfläche 11 umgebenen Öffnung 69 herausragt. Der dabei resultierende Überstand wird durch die Längsposition der Stellschraube 89, d.h. des an ihr ausgebildeten Anschlags 17b relativ zu dem Anschlag 10b des Referenzelementes, bestimmt. Bei Kontakt dieser beiden Anschläge wird das Referenzelement gegen die Kraft einer Rückholfeder 93 mit dem Nadelelement 6 synchron mitgeführt, bis der Einstich erfolgt, dessen Tiefe durch den Überstand der Nadelspitze 13 gegenüber der Hautkontaktfläche 11 bestimmt ist.

Die Bauelemente 88,89 und 10 sind bei dieser Ausgestaltung Bestandteile des Magazins, die jedoch an jedem Magazin nur einmal vorhanden sind. Alternativ können sie auch als Bestandteil des Stechgerätes realisiert sein. Jedenfalls ist es vorteilhaft, wenn bei einem erfindungsgemäßen Stechsystem mit magazinierten Stecheinheiten für alle Stecheinheiten des Magazins nur ein (gemeinsames) Stechtiefenreferenzelement vorgesehen ist. Dabei findet zwischen den Stechvorgängen eine Transportbewegung statt, mittels der jeweils ein neues Nadelelement 6 in die (in Figur 17 dargestellte) Position gebracht wird, in der es mindestens während eines Teils der Stechbewegung mit dem Nadelelement bewegt wird, um die gewünschte Stechtiefe zu gewährleisten.

Die Erfindung kann in Verbindung mit unterschiedlichen Magazinkonstruktionen verwendet werden. Hierzu gehören insbesondere auch Trommelmagazine, wie sie beispielsweise in der erwähnten US 2004/0260325 beschrieben werden.

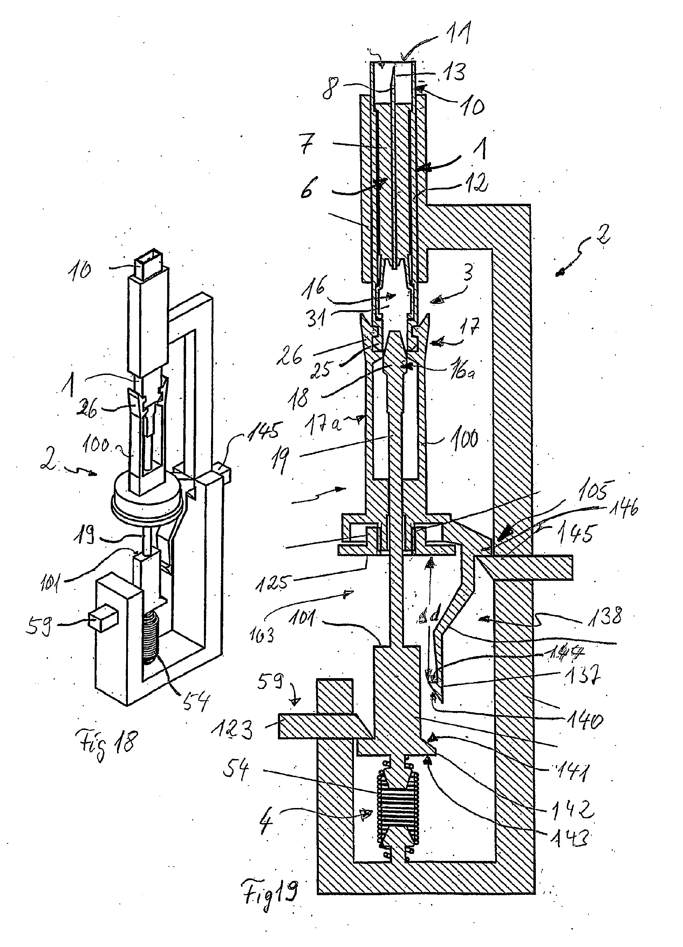

Bei dem in den Figuren 18 und 19 dargestellten Stechsystem stimmt die Gestaltung der Stecheinheit 1 und deren Verbindung mit dem Stechantrieb 4 weitgehend mit den Figuren 1 und 2 überein. Die Stecheinheit 1 besteht aus einem Nadelelement 6 mit einer Nadel 8 und einem die Nadel umschließenden Nadelelementkörper 7 aus Kunststoff sowie einem Stechtiefenreferenzelement 10 mit einem in diesem Fall hülsenförmigen Referenzelementkörper 12 und einer Hautkontaktfläche 11.

Die Verbindung mit dem Lanzettenantrieb 4 wird mittels eines Kopplungsmechanismus 3 hergestellt, der eine Nadelelementkupplung 16 und eine Referenzelementkupplung 17 einschließt. Übereinstimmend mit den Figuren 1 und 2 wird das Positionierungsteil 16a der Nadelelementkupplung 16 von dem Kopf 18 einer Verbindungsstange 19 gebildet, der in eine entsprechende Aufnahme 31 des Nadelelementkörpers 7 eingreift. Die Referenzelementkupplung ist auch in diesem Fall mittels einer aus Rasthaken 25 und Rastprofil 26 bestehenden Rastkupplung gestaltet, die an dem Stechtiefenreferenzelement 10 bzw. einem auf der Verbindungsstange 19 in Längsrichtung beweglich geführten Referenzelementhalter 100 ausgebildet wird.

Das Stechsystem der Figuren 18 und 19 hat (insofern übereinstimmend mit Figur 4) einen sogenannten "ballistischen" Stechantrieb 4, bei dem die Stechbewegung nicht in dem oben angegebenen Sinn zwangsgeführt ist. Bei einem ballistischen Stechantrieb ist die Stechbewegung mindestens in der Umgebung des Umkehrpunktes, vorzugsweise auf dem gesamten Bewegungsweg nur von der beschleunigenden Kraft einer oder mehrerer Antriebsfedern, der Massenträgheit der von diesen beschleunigten Bauteile und die Bewegung steuernden oder begrenzenden Anschlägen (sowie selbstverständlich der Reibung zwischen sich bewegenden Bauteilen) bestimmt.

Als Antriebsfeder 54 dient in dem dargestellten Fall eine Schraubenfeder, die in den Figuren im komprimierten Zustand dargestellt ist. Sie ist mit einer Verbindungsstange 19 gekoppelt, die von der Antriebsfeder 54 in Einstichrichtung beschleunigt wird, sobald ein Triggerlement 59 betätigt wird. Bei der dargestellten Ausführungsform ist das Triggerelement 59 ein Riegel 123, der in der dargestellten Halteposition die Verbindungsstange 19 arretiert. Zum Auslösen der Stechbewegung wird der Riegel 123 so zurückgezogen, daß die Verbindungsstange 19 von der Antriebsfeder 54 beschleunigt werden kann.

Die Referenzelementkupplung 17 wird beim Einsetzen der Stecheinheit 1 geschlossen. Die Nadelelementkupplung 16 wird hingegen erst nach der Betätigung des Triggerelementes geschlossen, wenn sich die Verbindungsstange 19 nach vorne bewegt. Von diesem Zeitpunkt an folgt sowohl die Bewegung des Nadelelementes 6, als auch die Bewegung des Stechtiefenreferenzelementes 10 exakt der Bewegung der entsprechenden Positionierungsteile 16a,17a, d.h. der Verbindungsstange 19 bzw. des Referenzelementhalters 100.

Während der weiteren Vortriebsphase bewegt sich die Verbindungsstange 19 nach vorne, bis sie mit einer Anschlagfläche 101 eine entsprechende Anschlagfläche 125 einer Einstelleinrichtung 126 kontaktiert, die am hinteren Ende des Referenzelementhalters 100 ausgebildet ist. Die Anschlagfläche 125 befindet sich an dem Kopf einer Einstellschraube 127, die auf ein in Einstichrichtung verlaufenden Gewinde 128 aufgeschraubt ist. Durch Drehen der Einstellschraube 127 kann die Längsposition der Anschlagfläche 125 relativ zu dem Referenzelement 10 (und damit der Hautkontaktfläche 11) eingestellt werden.

Bei der dargestellten bevorzugten Ausführungsform ist somit der Stechantrieb 4 direkt nur mit dem Nadelelement 6 gekoppelt, während das Referenzelement 10 über eine in der Vortriebsphase der Stechbewegung wirkende Mitnehmereinrichtung mit dem Nadelelement 6 und dadurch indirekt mit dem Stechantrieb 4 gekoppelt ist. Bestandteile der insgesamt mit 103 bezeichneten Mitnehmereinrichtung sind die beiden Anschläge 101 und 125, die während der weiteren Vortriebsphase bis zu dem Erreichen des Umkehrpunktes der Stechbewegung derartig aneinander anliegen, das ihr relativer Abstand die Längsposition der Nadelspitze 13 relativ zu der Kontaktfläche 11 des Stechtiefenreferenzelementes 10 und damit die Stechtiefe definiert. Die Anschläge 101,125 werden deshalb als Stechtiefenbegrenzungsanschläge bezeichnet.

Bei Erreichen des Umkehrpunktes ist die Antriebsfeder 54 gestreckt, so daß die Verbindungsstange 19 wieder zurückgezogen wird und damit die Rückzugsphase der Stechbewegung beginnt. Im dargestellten Fall dient also die Federkraft der Antriebsfeder 54 sowohl während der Vortriebsphase als auch während der Rückzugsphase der Stechbewegung zum Beschleunigen der Stecheinheit.

Für die bevorzugte Funktion des dargestellten Stechsystems ist auch wichtig, daß das Referenzelement 10 während eines Teils der Vortriebsphase und besonders bevorzugt (wie noch erläutert wird) auch während eines Teils der Rückzugsphase mittels eines Referenzbasisteils 146 auf einem Referenzelementlager 105 ruht. Als Referenzbasisteil wird dabei ein Funktionselement verstanden, das direkt oder indirekt mit dem Referenzelement 10 verbunden ist und dessen Bewegungsweg nach hinten im Zusammenwirken mit dem Referenzelementlager in einer definierten Position limitiert. Bei der dargestellten Gestaltung wird es von einer Schulter gebildet, welche an einer Nadelelementrückführsperre 138 ausgebildet ist, die mit dem Referenzelementhalter 100 verbunden ist und deren Funktion noch näher erläutert wird.

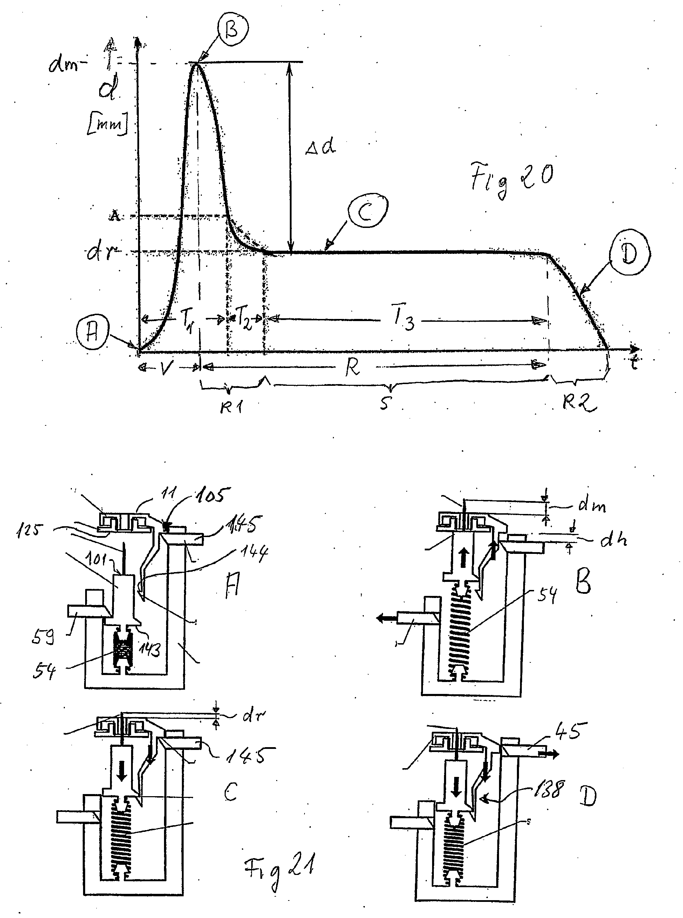

Bei der hier dargestellten Ausführungsform ist die Position des Referenzelementlagers 105 unter Berücksichtigung der Abmessungen der übrigen Bauteile so gewählt, daß der Kontakt der Referenzelementanschläge 101,125 erst kurz vor dem Umkehrpunkt der Stechbewegung stattfindet, so daß der Referenzelementhalter 100 bis zum Erreichen des Umkehrpunktes nur geringfügig von dem Referenzelementlager 105 abhebt. Selbst wenn sich das Referenzelement 10 frei in Einstichrichtung bewegen kann (also insbesondere nicht auf ein in seinem Bewegungsweg befindliches Körperteil auftrifft), beträgt der in Figur 21B dargestellte maximale Hub dh, um den das Referenzbasisteil 146 von dem Referenzelementlager 105 abhebt, höchstens 5 mm, bevorzugt höchstens 3,5 mm und besonders bevorzugt höchstens 2 mm.

Zu Beginn der Rückzugsphase wird das Referenzelement 10 gemeinsam mit dem Nadelelement 6 um die kurze Strecke dh nach hinten bewegt, um die das Referenzbasisteil 146 von dem Referenzelementlager 105 abgehoben wurde. Diese Rückwärtsbewegung wird einerseits durch die Elastizität einer Hautoberfläche, an der die Hautkontaktfläche 11 anliegt und andererseits durch die Rückwärtsbewegung der Verbindungsstange 19 und des Nadelelementes 6 bewirkt, wobei die Reibung zwischen diesen Elementen und dem Stechtiefenreferenzelement 10 bzw. dem Referenzelementhalter 100 eine ausreichende Kraftübertragung bewirkt.

Durch das Referenzelementlager 105 wird eine weitere Rückwärtsbewegung des Referenzelementhalters 100 und damit des Referenzelementes 10 gestoppt, während die Verbindungsstange 19 und damit das Nadelelement 6 durch die Kraft der Feder 54 weiter nach hinten gezogen wird, bis die Bewegung durch ein Sperrelement 137 der erwähnten Nadelelementrückführsperre 138 abgestoppt wird. Im dargestellten Fall umfaßt die Nadelelementrückführsperre 138 ein Federelement 139 in Form eines Federarms, der an seinem freien Ende das als Sperrklinke ausgebildete Sperrelement 137 trägt. Die Sperrklinke hat eine abgeschrägte Gleitfläche 140, an der während der Vortriebsphase der Stechbewegung eine entsprechende Gleitfläche 141 vorbeigleitet, die an einem an der Verbindungsstange 19 angeordneten Rastvorsprung 142 ausgebildet ist. Während dieses Vorbeigleitens gibt der Federarm 139 der Nadelelementrückführsperre 137 elastisch zur Seite nach. Sobald der Rastvorsprung 142 das Sperrelement 137 passiert hat, kehrt dieses wegen der von dem Federarm 139 aufgebrachten Federkraft wieder in seine ursprüngliche Position zurück, in der es die Verbindungsstange 19 und damit das Nadelelement 6 abstoppt, wobei eine Anschlagfläche 143 des Rastvorsprungs 142 der Verbindungsstange 19 gegen eine Anschlagfläche 144 des Sperrelementes 137 stößt.

Die Nadelelementrückführsperre 138 kann konstruktiv auch anders gestaltet sein. Allgemein eignet sich grundsätzlich jedes Element, durch das die Rückführbewegung der Stecheinheit 6 in einer definierten Längsposition gestoppt werden kann. Bevorzugt ist diese Längsposition relativ zu der durch das Referenzelementlager 105 vorgegebenen Längsposition der Hautkontaktfläche 11 so gewählt, daß die Spitze 13 des Nadelelementes 8 um eine definierte Reststechtiefe gegenüber der Ebene der Hautkontaktfläche 11 hervorsteht.

Die Figuren 18 und 19 verdeutlichen, daß als Bestandteil des Kopplungsmechanismus 3 zwischen dem Stechantrieb 4 und der Stecheinheit 1 ganz unterschiedliche Kupplungstypen verwendet werden. Die Nadelelementkupplung 18 koppelt während der Vortriebsphase der Stechbewegung den Stechantrieb 4 mit dem Nadelelement 6 und bildet danach eine bidirektional wirkende feste Verbindung zwischen diesen Elementen. Die Verbindung zwischen dem Stechantrieb 4 und dem Referenzelement 6 wird indirekt über den Referenzelementhalter 100 hergestellt. Dieser wird einerseits beim Einsetzen der Stecheinheit 1 mittels der bidirektional wirkenden Referenzelementkupplung 17 fest mit dem Referenzelement 10 verbunden. Andererseits ist der Referenzelementhalter 100 Bestandteil der Mitnehmereinrichtung 103, die zwei Paare von Anschlägen 125,101 und 143,144 einschließt, welche jeweils eine in eine Richtung wirkende (unidirektionale) Kupplung bilden, die so ausgebildet ist, daß die Verbindungsstange 19 und mit ihr das Nadelelement 6 über eine Strecke Δd zwischen den Anschlägen 125 und 144 relativ zu dem Referenzelement beweglich ist. Ad bildet vorzugsweise den Unterschied zwischen einer maximalen Einstichtiefe dm und einer Reststichtiefe dr, wie nachfolgend noch näher erläutert wird.

Durch die Konstruktion der Figuren 18 und 19 kann die in Figur 20 in Form eines Weg-Zeit-Diagramms ("Stechprofil") dargestellte Stechbewegung realisiert werden, die vor allem für Mikrosampler-Stechsysteme, allgemein für Stechsysteme, deren Nadelelement einen Kapillarkanal zur Aufnahme einer Probe aufweist, geeignet ist. Figur 21 zeigt vier Benutzungspositionen eines mit dem Stechsystem der Figuren 18 und 19 weitgehend übereinstimmenden Stechsystems, wobei einige Konstruktionselemente, die für die hier erläuterten Funktionen nicht bedeutsam sind, vereinfacht wurden. Die den Benutzungspositionen A bis D entsprechenden Zeitpunkte sind in Figur 20 mit diesen Buchstaben markiert.

In Figur 20 ist der zeitliche Verlauf der Einstechtiefe d eines Nadelelementes während der Vortriebsphase V und einer Rückzugsphase R dargestellt, wobei die Rückzugsphase R aus einem ersten Rückzugsabschnitt R1, einem Sammelabschnitt S und einem zweiten Rückzugsabschnitt R2 besteht. Die Zeitachse ist ungleichmäßig geteilt. Der mit T₁ bezeichnete Abschnitt der Bewegung läuft in der Praxis innerhalb von wenigen Millisekunden ab, während der mit T₂ bezeichnete Bewegungsabschnitt (Abbremsen vor dem Sammelabschnitt) einige hundert Millisekunden dauert und der Sammelabschnitt (T₃) mehrere Sekunden dauert.

Am Ende der Vortriebsphase V (Benutzungsposition B) erreicht das Stechelement eine maximale Einstechtiefe dm, die je nach Einstellung des Stechgerätes typischerweise 0,8 mm bis 2,3 mm beträgt. Daran schließt sich der erste Rückzugsabschnitt R1 an, in dem das Stechelement um eine Rückzugsstrecke Δd teilweise zurückgezogen und gegen Ende abgebremst wird, so daß es mit einer vorgegebenen Reststechtiefe dr von beispielsweise 0,5 mm in die Haut hineinragt (Benutzungsposition C). Schließlich folgt ein zweiter Rückzugsabschnitt R2 (Benutzungsposition D), in dem die Nadel 8 des Nadelelements 6 vollständig aus der Haut herausgezogen wird.

Bei der in den Figuren 19,20 und 21 gezeigten Konstruktion wird der zweite Rückzugsabschnitt am Ende der Sammelphase dadurch eingeleitet, daß der Sperriegel 145 aus der in den Figuren 19 und 21A bis 21C dargestellten Eingriffsposition in eine in Figur 21D gezeigte Rückzugsposition gezogen wird, so daß die Rückführsperre 138 zusammen mit der Verbindungsstange 19 von der Antriebsfeder 54 nach hinten bewegt wird. Dadurch wird die Rückzugsphase abgeschlossen und die Nadel 8 des Nadelelementes 6 vollständig aus der Haut entfernt.

Vergleicht man die in den Figuren 15 und 20 dargestellten Stechprofile, so besteht ein erster Unterschied darin, daß sich die Reststichtiefe dr während des Sammelabschnitts S der Rückzugsphase praktisch nicht ändert, sondern - bei der in den Figuren 18 bis 21 dargestellten Konstruktion durch das Zusammenwirken des Referenzelementlagers 105 und der Nadelelementrückführsperre 138 - konstant vorgegeben ist. Besonders bedeutsam ist aber, daß die maximale Stechtiefe dm unabhängig von der Reststechtiefe dr eingestellt werden kann. Insoweit unterscheidet sich das Stechsystem der Figuren 18 bis 21 von den zuvor beschriebenen Stechsystemen, bei denen sich die Einstellung der Stechtiefe auf die gesamte Stechbewegung auswirkt und sich deswegen die Reststechtiefe dr, bei der die Probe in einen Mikrosampler aufgenommen wird, unvermeidlich verändert, wenn man die maximale Stechtiefe dm verstellt.

Im Rahmen der Erfindung wurde festgestellt, daß es für die Qualität der Probengewinnung und für das Schmerzempfinden bei der Benutzung von Mikrosampler-Stechsystemen sehr vorteilhaft ist, wenn die hier als Reststechtiefe bezeichnete Stechtiefe, mit der die Nadel des Nadelelementes während des Sammelns der Probe in die Haut hineinragt, unabhängig von der eingestellten maximalen Stechtiefe näherungsweise konstant ist. Geringe Schwankungen der Reststechtiefe oder eine langsame Bewegung des Nadelelementes während der Aufnahme der Probe sind zwar akzeptabel. Erforderlich ist jedoch, daß die maximale Stechtiefe dm unabhängig von dem zeitlichen Mittel der Reststechtiefe während der Aufnahme der Probe eingestellt werden kann.

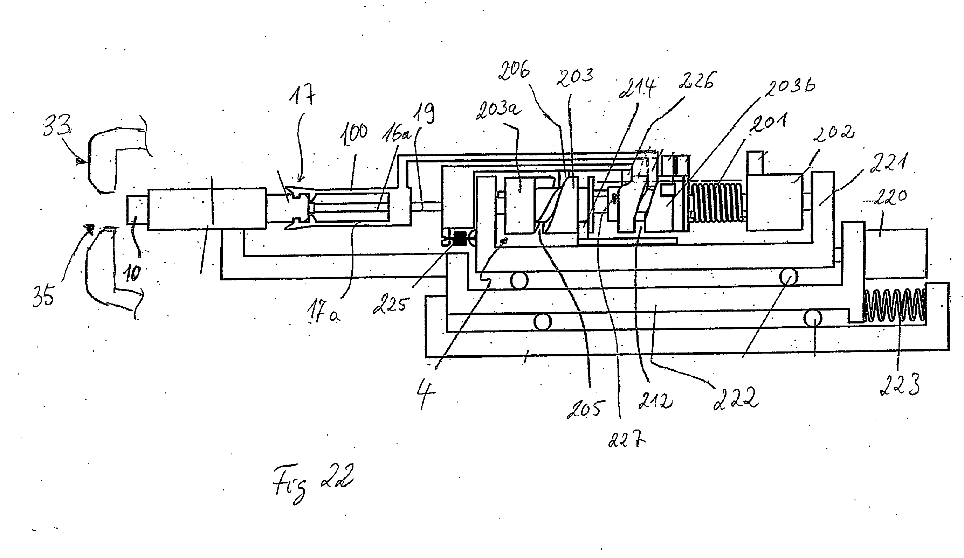

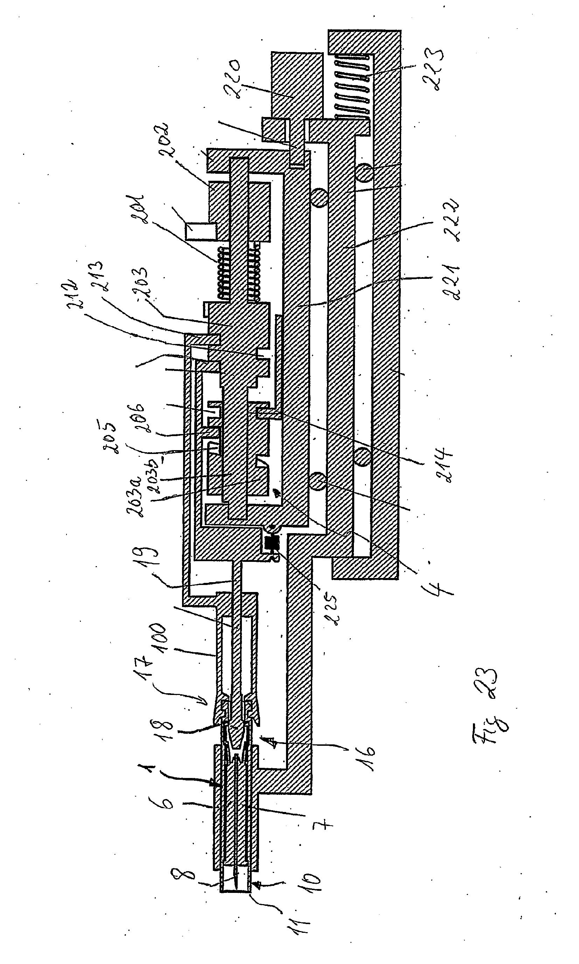

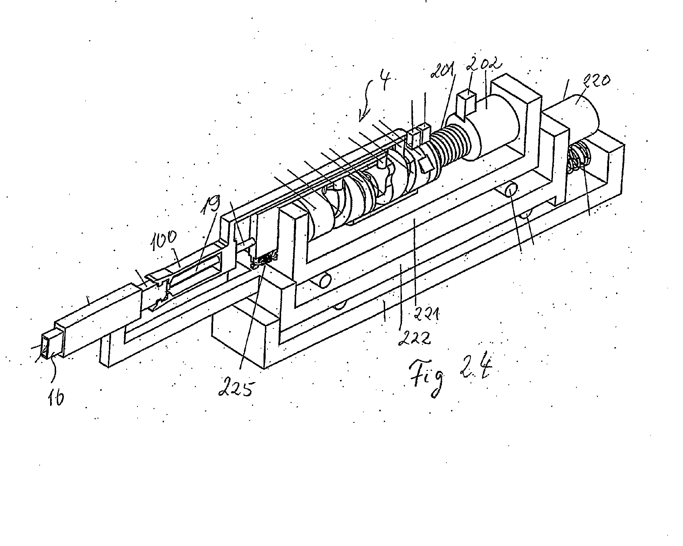

Ein weiteres Ausführungsbeispiel eines Stechsystems, mit dem sich ein Stechprofil mit den anhand von Figur 20 erläuterten Besonderheiten verwirklichen läßt, ist in Figur 22 in Seitenansicht, in Figur 23 im Querschnitt und in Figur 24 in einer perspektivischen Schrägansicht dargestellt.

Die Gestaltung der Stecheinheit 1 und der Kupplungen 16 und 17 stimmt mit den Figuren 18 und 19 überein. Diese Elemente, die auch hier mit den gleichen Bezugsziffern bezeichnet sind und deren Funktion werden deshalb nicht nochmals beschrieben.

Hingegen unterscheidet sich der Stechantrieb 4 wesentlich von den Figuren 18 und 19. Es handelt sich um einen Rotorantrieb mit Hubausgleich, der nachfolgend näher erläutert wird.

Zu dem Stechantrieb 4 des dargestellten Stechgerätes 2 gehört eine Antriebsfeder 201, ein Spannrotor 202 zum Spannen der Antriebsfeder und ein von der Antriebsfeder 201 angetriebene Antriebsrotor 203. Die Drehbewegung des Antriebsrotors 203 wird mittels einer Steuereinrichtung in Form einer Kurvensteuerung in die Stechbewegung des Nadelelementes 6 umgesetzt. Eine Nadelelement-Steuerkurve 205 ist als Nut in dem Antriebsrotor 203 ausgebildet und wird von einem Nadelelement-Steuerkurvenreiter 206 (in Form eines in die Steuerkurve 205 eingreifenden Zapfens) abgefahren, der mit einer Verbindungsstange 19 verbunden ist, mit welcher das Nadelelement 6 gekoppelt ist.

Die Bewegung des Stechtiefenreferenzelementes 10 wird ebenfalls durch eine Kurvensteuerung, bestehend aus einer Referenz-Steuerkurve 212 und einem Referenz-Steuerkurvenreiter 213 gesteuert. Die Referenz-Steuerkurve 212 ist ebenfalls als Nut in dem Antriebsrotor 203 ausgebildet. In die Steuerkurve 212 greift ein Steuerkurvenreiter 213 ein, der mit dem Referenzelementhalter 100 verbunden ist.

Zum Einstellen der Stechtiefe ist der Abstand der beiden Steuerkurven 205 und 212 mittels einer Einstelleinrichtung 214 in Form einer justierbaren Axiallagerung einstellbar. Die beiden Steuerkurven 205 und 212 sind auf einem ersten Teil 203a des Antriebsrotors 203 bzw. auf einem zweiten Teil 203b des Antriebsrotors 203 angeordnet. Mittels der Einstelleinrichtung 214 kann der Abstand zwischen dem ersten Teil 203a und dem zweiten Teil 203b des Antriebsrotors 203 variiert werden.

Bei der Benutzung wird das Stechgerät mit einer nur in Figur 21 schematisch angedeuteten, eine Gehäuseöffnung 35 umschließenden Gehäuse-Hautkontaktfläche 33 an eine Hautoberfläche des Benutzers angedrückt. Anschließend wird zum Zweck eines (aktiven) Hubausgleichs der Abstand des Referenzelementes 10 von der Hautoberfläche bestimmt. Dazu wird mittels eines Elektromotors 220 ein in Einstichrichtung verschiebbarer erster Schlitten 221 an die Hautoberfläche herangefahren. Auf dem ersten Schlitten 221 ist der Antrieb 4 einschließlich des von der Verbindungsstange 19 gebildeten Nadelelement-Positionierungsteils 16a und des von dem Referenzelementhalter 100 gebildeten Referenzelement-Positionierungsteils 17a gelagert. Der Schlitten 221 wird nach vorne bewegt, bis das Referenzelement 10 die Haut berührt. Dies kann elektronisch, beispielsweise durch eine induktive oder kapazitive Messung, festgestellt werden. Anschließend wird der Schlitten 221 wieder etwas zurückgefahren, bis ein definierter Abstand zu der Hautoberfläche besteht.

Nach dem Auslösen einer Stechbewegung werden das NadelelementPositionierungsteil 16a und das Referenzelement-Positionierungsteil 17a während der Vortriebsphase der Stechbewegung durch eine ansteigende Flanke der entsprechenden Steuerkurven 205, 212 nach vorn geschoben. Während des Eindringens der Nadel 8 des Nadelelementes 6 in die Hautoberfläche liegt das Referenzelement 10 mit seiner Hautkontaktfläche 11 an dieser an, so daß ein Referenzpunkt für eine präzise Einstichtiefe definiert ist.

Zum Ausgleich der Z-Varianz ist auch bei dieser Ausgestaltung ein Hubausgleich 38 vorgesehen, wobei hier sogar zwei Hubausgleich-Konstruktionen in Kombination verwendet werden:
- Einerseits ist ein aktiver Hubausgleich dadurch realisiert, daß die Position der Hautoberfläche vor dem Auslösen einer Stechbewegung detektiert und die nachfolgende Stechbewegung an die zuvor detektierte Position (hier durch Verschieben der gesamten Hubstrecke) angepaßt wird. Diese Form des aktiven Hubausgleichs ist nicht nur in der dargestellten Ausgestaltung, sondern auch in anderen konstruktiven Realisierungen vorteilhaft.
- Zusätzlich ist ein passiver Hubausgleich vorgesehen, der hier dadurch realisiert ist, daß der erste Schlitten 221 in Längsrichtung verschiebbar auf einem zweiten Schlitten 222 gelagert ist, welcher gegen die Kraft einer Andruckkontrollfeder 223 zurückgeschoben werden kann. Dadurch wird eine maximale Andruckkraft definiert, die über das Referenzelement 10 auf die Hautoberfläche wirken kann. Dieser zusätzliche passive Hubausgleich ist fakultativ.

Nach Erreichen des Umkehrpunktes der Stechbewegung werden das Nadelelement-Positionierungsteil 16a und das Referenzelement-Positionierungsteil 17a zurückgezogen. Die auf das Nadelelement-Positionierungsteil wirkende Kurvensteuerung 205, 206 hat die Besonderheit, daß sich der Nadelelement-Steuerkurvenreiter 206 bei der Rückführbewegung aus seinem Eingriff in die entsprechende Steuerkurve 205 löst. Dies bedeutet, daß der Steuerkurvenreiter nicht, wie sonst üblich, in der gesamten Steuerkurve so zwangsgeführt ist, daß jeder Position des Steuerkurvenreiters auf der Steuerkurve eine definierte Längsposition des Reiters und damit des von diesem gesteuerten Elementes (Nadelelement oder Referenzelement) in Einstichrichtung entspricht. Vielmehr ist die Längsposition des Steuerkurvenreiters und damit des gesteuerten Elementes bei gelöstem Eingriff der Steuerkurve allenfalls in einer Raumrichtung (in Einstichrichtung oder entgegen der Einstichrichtung) begrenzt, mindestens in der entgegengesetzten Raumrichtung jedoch frei. Dies wird bei der dargestellten Ausgestaltung dadurch erreicht, daß die die Steuerkurve 205 bildende Nut so weit verbreitert ist, daß der Steuerkurvenreiter 206 dort nicht mehr geführt wird. Das Nadelelement-Positionierungsteil 16a (und damit das Nadelelement) wird deshalb während der Rückzugsphase nicht aktiv von dem Nadelelement-Steuerkurvenreiter 206 zurückgezogen.

Die Rückführbewegung des Nadelelement-Positionierungsteils 16a wird statt dessen mittels einer Rückstellfeder 225 bewirkt. Sie koppelt das Nadelelement-Positionierungsteil 16a mit dem ersten Schlitten 221 und damit auch mit dem Antrieb 4. Während der Rückzugsphase wird das Nadelelement-Positionierungsteil 16a deshalb von der Rückstellfeder 225 relativ zu dem Antrieb nach hinten bewegt, bis es mit einem zweiten Steuerkurvenreiter 226 an eine weitere Steuerkurve 227 stößt, die auf dem zweiten Teil 203b des Antriebsrotors 203 angebracht ist. Die weitere Steuerkurve 227 bildet also eine Rückführsperre, mit der die Bewegung des Nadelelementes 6 am Ende des ersten Rückzugsabschnitts R1 abgestoppt wird. Dadurch ist eine definierte Lage des Nadelelement-Positionierungsteils 16a gegenüber dem Referenzelement-Positionierungsteils 17a gegeben, bei der die Spitze 13 der Nadel 8 mit einer vorgegebenen Reststechtiefe über die Hautkontaktfläche 11 des Stechtiefenreferenzelementes 10 hinausragt. Nach Abschluß des Sammelabschnitts S wird das Nadelelement 6 vollständig aus der Haut herausgezogen, indem der erste Schlitten 221 mit dem Elektromotor 220 zurückgefahren wird.

Die in den Figuren 17, 18/19 und 22 bis 24 dargestellten Ausführungsformen zeigt beispielhaft, daß das Stechtiefenreferenzelement 10 nicht (wie beispielsweise bei den Figuren 1 und 2) während der gesamten Vortriebsphase gemeinsam mit dem Nadelelement 6 bewegt werden muß. Vielmehr kann es vorteilhaft sein, wenn das Stechtiefenreferenzelement 10 nur in dem dem Umkehrpunkt unmittelbar vorausgehenden Teil der Vortriebsphase gemeinsam (d.h. gleichzeitig, nicht notwendigerweise gleich schnell) mit dem Nadelelement bewegt wird. Vorzugsweise beträgt der Hub (Wegstrecke in Einstichrichtung), um den das Stechtiefenreferenzelement 10 bis zum Erreichen des Umkehrpunktes gemeinsam mit dem Nadelelement 6 bewegt wird, höchstens 5 mm, vorzugsweise höchstens 3,5 mm und besonders bevorzugt höchstens 2 mm. Bei der in den Figuren 17 und 18/19 dargestellten Konstruktion wird dies dadurch realisiert, daß das Stechtiefenreferenzelement 10 in einer Ruheposition auf einem Referenzelementlager ruht und ausgehend von dieser Ruheposition von der Bewegung des Nadelelementes mitgenommen wird, kurz bevor das Nadelelement den Umkehrpunkt erreicht. Andere Konstruktionen sind jedoch möglich, wie die Figuren 22 bis 24 zeigen.

Weiterhin ist es vorteilhaft, wenn der Teilabschnitt der Stechbewegung, während dessen das Stechtiefenreferenzelement gemeinsam mit dem Nadelelement während der Vortriebsphase (also bis zum Erreichen des Umkehrpunktes) bewegt wird, in sehr kurzer Zeit zurückgelegt wird. Vorzugsweise sollte diese Zeitdauer maximal 100 msec, bevorzugt maximal 50 msec und besonders bevorzugt maximal 10 msec betragen.

Im Rahmen der Erfindung wurde festgestellt, daß durch die vorstehend genannten Maßnahmen, die einzeln oder in Kombination eingesetzt werden können, einerseits die Stechtiefe präzise kontrolliert werden kann, andererseits die viskoelastischen Eigenschaften der Haut bei einem derartig kurzen Anpressen nicht dazu führen, daß die präzise Definition der Reststechtiefe durch eine Verformung der Haut beeinträchtigt wird. Ergänzend ist es vorteilhaft, wenn bei einem Stechprofil der in Figur 20 dargestellten Art der erste Rückzugsabschnitt R1 zwischen dem Erreichen des Umkehrpunktes (maximale Stechtiefe) und dem Beginn des Sammelabschnitts maximal 2 Sekunden, bevorzugt maximal 1 Sekunde und besonders bevorzugt maximal 0,5 Sekunden dauert.

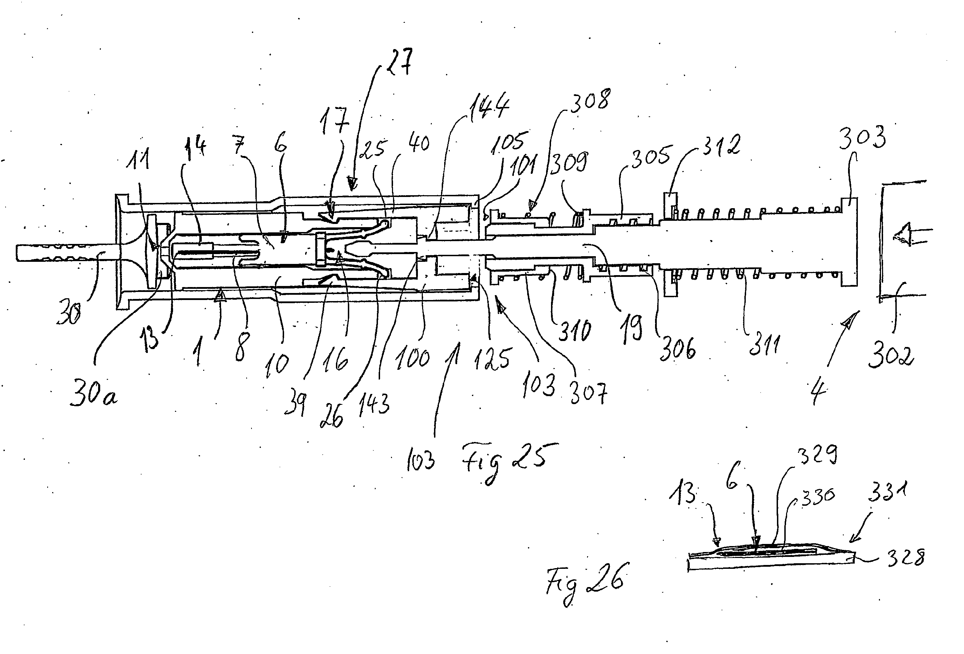

Bei dem in Figur 25 dargestellten Stechsystem stimmt die Gestaltung der Stecheinheit und der Kupplungen 16 und 17 im wesentlichen mit den Figuren 18/19 und 22 bis 24 überein. Diese Elemente werden wiederum mit den gleichen Bezugsziffern bezeichnet und nicht nochmals beschrieben.

Ebenfalls übereinstimmend mit den Figuren 18 und 19 wird das Referenzelementpositionierungsteil 17a von einem Referenzelementhalter 100 gebildet, der in dem nicht dargestellten Gehäuse in Längsrichtung beweglich ist und in der dargestellten Ausgangsposition der Stechbewegung auf einem Referenzelementlager 105 ruht. Der Referenzelementhalter 100 und eine mit diesem über die Referenzelementkupplung 17 verbundene Stecheinheit 1 sind in einem Gehäuseteil 301 in Längsrichtung geführt. Das übrige Gehäuse ist nicht dargestellt. Auch bei diesem Stechsystem ist jedoch eine Gehäuse-Hautkontaktfläche vorhanden, wie sie beispielhaft in den Figuren 2c und 22 dargestellt ist.

Eine weitere Gemeinsamkeit mit den Figuren 18 und 19 besteht darin, daß ein ballistischer Stechantrieb 4 verwendet wird. Er arbeitet im dargestellten Fall jedoch nach dem "Hammer-Amboß-Prinzip", d.h. ein als Hammer 302 bezeichnetes in Figur 25 nur symbolisch dargestelltes Bauteil wird schnell in Richtung auf einen Amboß 303 bewegt, der seinerseits über einen Kopplungsmechanismus 3 mit der Stecheinheit 1 in Wirkverbindung steht.

Auch bei dieser Ausführungsform wird die Referenzelementkupplung 17 beim Einsetzen der Stecheinheit 1 geschlossen. Der Referenzelementhalter 100 bildet dabei eine Halterung 27 zur Aufnahme einer disposiblen Stecheinheit 1. Die Nadelelementkupplung 16 wird erst geschlossen, wenn die mit dem Amboß 303 verbundene Verbindungsstange 19 nach vorne bewegt wird. Eine weitere Übereinstimmung mit den Figuren 18 und 19 besteht darin, daß auch hier eine Mitnehmereinrichtung 103 vorgesehen ist, zu der zwei korrespondierende Anschläge 101 und 125 gehören. Auch hier ist der Stechantrieb 4 direkt nur mit dem Nadelelement 6 gekoppelt, während das Referenzelement 10 über die in der Vortriebsphase der Stechbewegung wirkende Mitnehmereinrichtung 103 mit dem Nadelelement 6 und dadurch indirekt mit dem Stechantrieb 4 gekoppelt ist.

Abweichend von den Figuren 18 und 19 ist bei Figur 25 die Stechtiefeneinstellung durch eine Longitudinalverschiebung eines mit dem Nadelelement 6 (nicht mit dem Referenzelement 10) verbundenen Stechtiefenbegrenzungsanschlags realisiert. Bei der dargestellten Ausführungsform ist ein Stechtiefenverstellring 305 auf einem Gewinde 306 der Verbindungsstange 19 drehbar und kann dadurch in seiner Längsposition relativ zu dem Nadelelement 6 verstellt werden. Der Stechtiefenverstellring 305 dient zur elastischen Lagerung eines auf der Verbindungsstange 19 in Longitudinalrichtung verschiebbaren Anschlagrings 307, wobei die elastische Lagerung durch eine aus Metall bestehende Andruckfeder 308 bewirkt wird.

Wenn unter Einwirkung des Hammers 302 der Amboß 303 nach vorne (in Figur 25 nach links) bewegt wird, schließt die bidirektional wirkende Nadelelementkupplung 16. Wenn sich die Anschlagflächen 101 und 125 berühren, bilden sie eine unidirektional wirkende Kupplung, durch die eine elastisch gefederte Verbindung zwischen der Verbindungsstange 19 (und damit dem Antrieb 4) und dem Referenzelement 10 bewirkt wird. Die Longitudinalabmessungen der Bauteile sind so aufeinander abgestimmt, daß in der anschließenden Bewegungsphase bis zu dem Kontaktierungszeitpunkt (Auftreffen der Hautkontaktfläche des Stechtiefenreferenzelementes auf die Hautoberfläche) der Abstand zwischen der Hautkontaktfläche 11 und der Spitze 13 des Nadelelementes 6 kleiner als der gewünschte (vorgegebene) Wert der Stechtiefe ist. Vorzugsweise befindet sich in dieser Bewegungsphase die Spitze 13 des Nadelelementes 6 sogar in Einstichrichtung hinter der Hautkontaktfläche, so daß der Einstich in die Haut erst nach dem Kontaktierungszeitpunkt erfolgt.

Bei Kontaktierung der Haut wird das Referenzelement 10 gestoppt. Die Elastizität der Andruckfeder 308 (allgemeiner gesagt die Elastizität der Kopplung zwischen dem Stechtiefenreferenzelement 10 und dem Stechantrieb 4) stellt sicher, daß die Hautkontaktfläche 11 mit einem der Federkonstante entsprechenden Druck gegen die Haut gedrückt wird und diese strafft, während eine weitere Bewegung des Nadelelementes nach vorne möglich ist, bis der Umkehrpunkt der Stechbewegung erreicht ist.

Die erforderliche Begrenzung der Bewegung des Nadelelementes wird bei der dargestellten Ausführungsform dadurch erreicht, daß die Relativbewegung zwischen dem Stechtiefenverstellring 306 und dem Anschlagring 307 gestoppt wird, wenn an diesen Elementen vorgesehene Anschläge 309 bzw. 310 aufeinandertreffen. Dadurch wird der maximale Bewegungsweg des Nadelelementes 6 relativ zu dem Referenzelement 10 begrenzt und erreicht, daß das Nadelelement 6 am Umkehrpunkt der Stechbewegung eine definierte Längsposition in Einstichrichtung relativ zu dem Stechtiefenreferenzelement 10 hat. Demzufolge wirken auch die Anschläge 309 und 310 als Stechtiefenbegrenzungsanschläge. Die Anschläge 309 und 310 sowie die Anschläge 101 und 125 bilden zwei unidirektional wirkende Kupplungen und liegen am Umkehrpunkt der Stechbewegung derartig aneinander an, daß ihre relativen Abstände zueinander die Stechtiefe definieren.

Die Rückzugsphase der Stechbewegung wird durch eine Rückholfeder 311 angetrieben, die sich einerseits gegen ein gehäusefestes Lagerteil 312 und andererseits gegen den Amboß 303 abstützt. Zunächst wird das Nadelelement 6 nach hinten gezogen, wobei die Nadelspitze 13 hinter die Hautkontaktfläche 11 zurückgezogen wird und die Rasthaken 25 erneut in die Rastprofile 26 eintreten, so daß sie die Längsposition des Nadelelementes 6 in dem Stechtiefenreferenzelement 10 fixieren. Die weitere Rückwärtsbewegung der Verbindungsstange 19 wirkt auf die gesamte Stecheinheit 1, bis sie durch den Kontakt des Referenzelementhalters 100 mit dem Referenzelementlager 105 gestoppt wird.

In der dargestellten Endposition ist das Stechtiefenreferenzelement 10 über ein Paar von korrespondierenden Anschlägen 143,144 mit dem Stechantrieb 4 gekoppelt. Auch bei Figur 25 wirkt die Mitnehmereinrichtung 103 bidirektional einerseits durch die Anschläge 101,125, 309,310 und andererseits durch die Anschläge 143 und 144. Anders als bei Figur 18 und 19 wird dadurch jedoch nicht eine definierte Reststichtiefe sichergestellt, sondern durch die in der Rückzugsphase wirkenden Anschläge 143,144 wird der Referenzelementhalter 100 und mit ihm das Referenzelement in der dargestellten Position festgehalten. Die benutzte Stecheinheit 1 kann insgesamt mittels eines hier nicht dargestellten Auswerfers aus der Halterung 100 herausgedrückt und ausgeworfen werden.

Die praktische Erprobung der in Figur 25 dargestellten Ausführungsform der Erfindung hat ergeben, daß damit eine sehr gut reproduzierbare Stechtiefe und ein extrem geringes Schmerzempfinden erreicht wird. Nach dem gegenwärtigen Kenntnisstand der Erfinder ist dies unter anderem darauf zurückzuführen, daß die Hautkontaktfläche des Stechtiefenreferenzelementes einen zur Straffung der Haut ausreichenden Druck auf die Hautoberfläche ausübt, wenn der Einstich erfolgt. Deswegen sollte der Stechantrieb und der Kopplungsmechanismus so ausgebildet sein, daß zum Hautkontaktierungszeitpunkt (Zeitpunkt des Kontaktes der Hautkontaktfläche 11 mit der Hautoberfläche) die Spitze 13 des Nadelelementes 8 nicht oder nur so geringfügig gegenüber der Hautkontaktfläche hervorsteht, daß zum Zeitpunkt des Eintretens der Nadelspitze in die Haut der Druck der Hautkontaktfläche bereits wirksam ist.

Im Rahmen der Erfindung wurde festgestellt, daß die Longitudinalposition des Nadelelementes 8 und der Hautkontaktfläche 11 sowie der von der Hautkontaktfläche 11 (während des folgenden Teilabschnitts der Vortriebsphase) auf die Hautoberfläche ausgeübte Druck experimentell so optimiert werden können, daß dadurch der Einfluß einer mit dem Einstechen des Nadelelementes in die Haut verbundenen Hautdeformation auf die Reproduzierbarkeit der Stechtiefe entscheidend vermindert wird. Experimente haben gezeigt, daß dies so gut möglich ist, daß die Hautdeformation keinen in der Praxis störenden Einfluß auf die Stechtiefe mehr hat. Dies zeigt sich auch daran, daß die tatsächliche Tiefe des Stichs in der Haut sehr gut mit der eingestellten Stechtiefe des Gerätes (Abstand zwischen der Spitze 13 und der Hautkontaktfläche 11) übereinstimmte, während ohne die hier beschriebenen Maßnahmen festgestellt wurde, daß die tatsächliche Stichtiefe in der Haut um mehr als 0,3 mm kleiner als die am Gerät eingestellte Stechtiefe war.

Dabei spielt auch die Größe der Hautkontaktfläche eine Rolle. Ihr Durchmesser sollte mindestens 1,5mm, vorzugsweise aber nicht mehr als 6mm betragen. Die Hautkontaktfläche 11 muß möglichst kantenfrei (englisch: "soft edged") ausgebildet sein. Insbesondere wäre es nachteilig, wenn beim Abnehmen der Einsetzhilfe 30 von dem Stechtiefenreferenzelement 10 eine scharfe Kante im Bereich der Hautkontaktfläche 11 entstünde, wie dies bei Verwendung der in diesem Zusammenhang gebräuchlichen Sollbruchstellen-Konstruktion (mit einer Schwachstelle aus dünnem Kunststoff) unvermeidlich ist. Im dargestellten Fall wird deshalb die Verbindung zwischen der Einsetzhilfe 30 und dem Stechtiefenreferenzelement 10 mittels eines Verbindungsprofils 30a aus weichem hochelastischem Kunststoff hergestellt. Es sind jedoch auch andere Konstruktionen möglich, sofern gewährleistet ist, daß die Hautkontaktfläche nach Entfernen der Einsetzhilfe 30 bruchkantenfrei ist. Bevorzugt ist eine Ausgestaltung der Hautkontaktfläche, bei der sie in der Umgebung der Nadelelement-Austrittsöffnung konvex gekrümmt ist.

Da der Effekt der Hautstraffung und der damit verbundenen ausgezeichneten Reproduzierbarkeit der Stechtiefe von einer Vielzahl von Faktoren abhängt, können keine allgemein verbindlichen Angaben über die diesbezüglich optimalen Parameter gemacht werden. Generell sollte jedoch der Druck, mit dem die Hautkontaktfläche 11 des Stechtiefenreferenzelementes 10 im letzten Teilabschnitt der Vortriebsphase gegen die Hautoberfläche drückt, so hoch sein, daß die mit dem Einstechen des Nadelelementes in die Haut verbundene Hautdeformation ("Eindellung") keinen praktisch störenden Einfluß auf die Reproduzierbarkeit der Stechtiefe hat. Dieser Druck sollte mindestens 1 N/cm², bevorzugt mindestens 3 N/cm² und besonders bevorzugt mindestens 5 N/cm² betragen. Im Rahmen der Erfindung wurde festgestellt, daß der aus diesem relativ starken Anpreßdruck resultierende Innendruck im Gewebe geeignet ist, negative Einflüsse, die die die Eindellung der Haut beim Einstechen der Nadel in die Hautoberfläche normalerweise hat, so zu minimieren, daß eine sehr gute Reproduzierbarkeit des Stechtiefe erreicht wird. Zugleich ergibt sich ein extrem geringes Schmerzempfinden.

Es ist davon auszugehen, daß auch die indirekte Ankopplung des Stechtiefenreferenzelementes 10 an den Antrieb 4 mittels der Mitnehmereinrichtung 103 und/oder die durch die metallene Andruckfeder 308 bewirkte elastische Kopplung und Druckkontrolle einen wesentlichen Beitrag zu der ausgezeichneten Funktion des dargestellten Stechsystems leistet. Die Feder ist vorgespannt derartig eingebaut, daß sie schon in ihrem maximal expandierten Zustand die gewünschte Mindestkraft überträgt. Die Kraftänderung je Längeneinheit (Federkonstante) beträgt vorzugsweise maximal 0,1 N/mm.

Die Mitnehmereinrichtung (insbesondere elastische Mitnehmereinrichtung) kann auch innerhalb der Stecheinheit selbst realisiert sein, wobei entsprechende Anschläge an den gegeneinander verschieblichen Elementen (Nadelelement und Stechtiefenreferenzelement) vorgesehen sein können. Im Falle einer elastischen Mitnehmereinrichtung kann dabei eine Feder vorgesehen sein, die sich einerseits an dem Nadelelement und andererseits an dem Stechtiefenreferenzelement abstützt.

Das in den Figuren 26 bis 30 dargestellte Stechsystem zeichnet sich vor allem dadurch aus, daß die hier vorzugsweise als einfache metallene nadelförmige Lanzetten 330 ausgebildeten Nadelelemente 6 mittels eines Nadelelementbandes 331 konfektioniert sind (Fig. 26). Das Nadelelementband 331 hat eine Tragfolie 328, auf der die Lanzetten 330 jeweils mit ihrem hinteren (von der Spitze 13 abgewandten) Ende klebend fixiert sind und eine die Lanzetten überdeckende und dadurch hygienisch versiegelnde Deckfolie 329. Beide Folien bestehen vorzugsweise aus Kunststoff. Die Konfektionierung von Lanzetten in Form von Bändern ist generell bekannt, beispielsweise aus der deutschen Patentschrift 28 03 345 und der EP 1 360 935 A1.

Im Zusammenhang mit der vorliegenden Erfindung ist wichtig, daß die Tragfolie 328 und die Deckfolie 329 des Nadelelementbandes 331 sehr dünn sind. Die Dicke der Tragfolie 328 beträgt vorzugsweise höchstens 100µm, besonders bevorzugt höchstens 50µm und noch weiter bevorzugt höchstens 30µm. Die Dicke der Deckfolie 329 sollte höchstens 50µm, bevorzugt höchstens 25µm und weiter bevorzugt höchstens 15µm betragen.

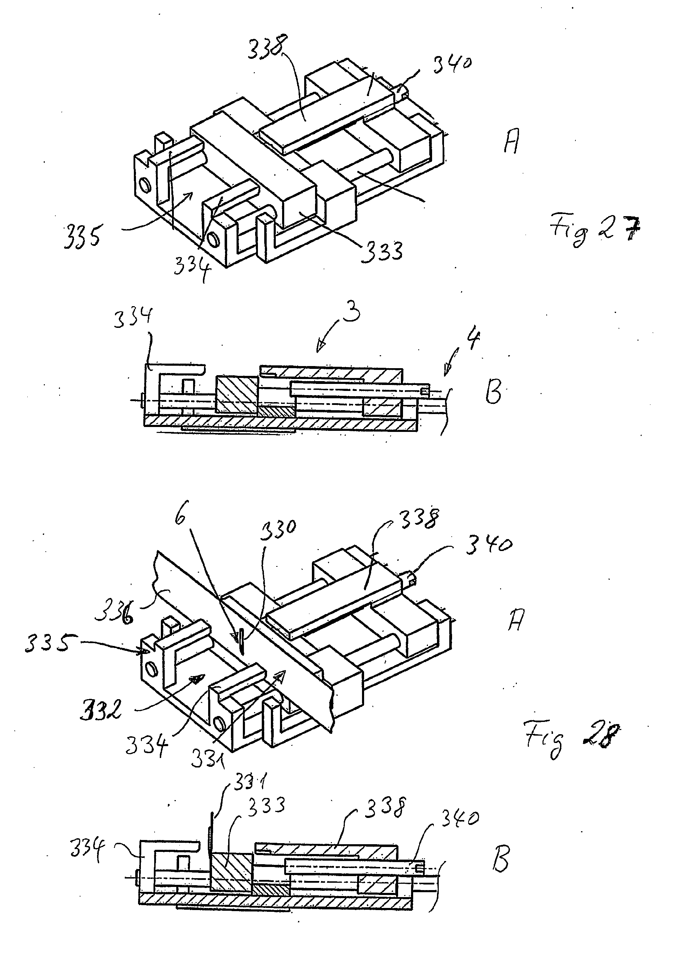

Im Rahmen der vorliegenden Erfindung wird das Nadelelementband 331 nicht nur dazu verwendet, eine Mehrzahl von Nadelelementen kostengünstig steril zu verpacken und auf einfache Weise der Benutzung zuzuführen. Vielmehr bildet die Tragfolie 328 im Rahmen der Erfindung während des Einstichs einen hygienischen Überzug im Bereich der Hautkontaktfläche und ist somit ein Bestandteil des Stechtiefenreferenzelementes. Eine geeignete Konstruktion des Stechsystems ist in vier Benutzungspositionen in den Figuren 27 bis 30 dargestellt.

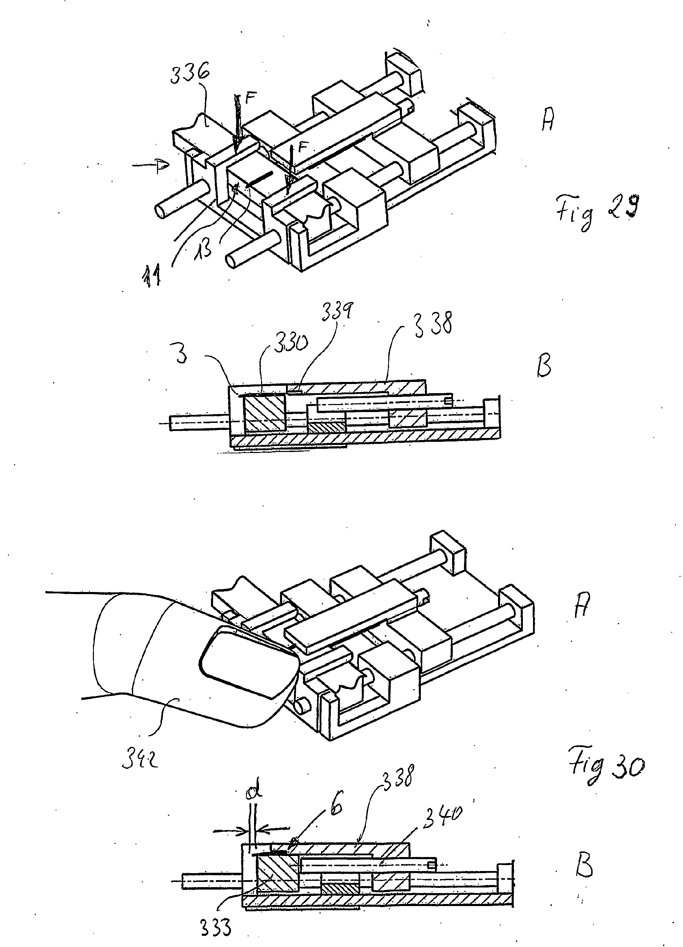

Das Nadelelementband 331 wird von einer nicht dargestellten Transporteinrichtung schrittweise so transportiert, daß sich jeweils eine Lanzette 330 an einer Stechstation 332 vor einem Referenzelementbasisteil 333 befindet (Figur 28). Ein Bandniederhalter 334 ist Bestandteil einer insgesamt mit 335 bezeichneten Stechvorbereitungseinrichtung, durch die ein an der Stechstation 332 befindliches Nadelelement 6 in eine Stechposition gebracht wird, bei der seine Spitze 13 freigelegt und in Einstichrichtung orientiert ist, während die Tragfolie 328 das Referenzelementbasisteil 333 in der Umgebung der Nadelspitze 13 abdeckt (Figur 29). Bei der dargestellten Ausführungsform ist der Bandniederhalter 334 in einer Ebene parallel zur Einstichrichtung L-förmig ausgebildet und er wird so über das Nadelelementband 331 geschoben, das es rechtwinklig abgeknickt wird, wobei ein Teil des Bandes die (in den Figuren linke) vordere Kante des Referenzelementbasisteils 333 abdeckt und dort die Hautkontaktfläche 11 bildet. Dies ist ein Beispiel für eine Ausführungsform der Erfindung, bei der nicht das gesamte Stechtiefenreferenzelement 10, sondern nur dasjenige Element, an dem die Hautkontaktfläche ausgebildet ist, zur einmaligen Verwendung vorgesehen (disposibel) ist.

Zur Bewegung jeweils einer Lanzette dient ein Lanzettengreifer 338, der von hinten (in den Figuren von rechts nach links) gegen die Lanzette bewegt wird, wobei eine Ausnehmung 339 des Lanzettengreifers das hintere Ende der Lanzette umschließt (Figur 30). Dabei ist der Bewegungsweg des Lanzettengreifers 338 relativ zu dem Referenzelementbasisteil 333 durch eine Stechtiefenjustierschraube 340 beschränkt. Wenn das vordere Ende der Stechtiefenjustierschraube 340 an dem Referenzelementbasisteil 333 anschlägt, wird bei weiterer Verschiebung des Lanzettengreifers 338 nach vorne (in den Figuren nach links) das Referenzelementbasisteil 333 und damit auch die Tragfolie 328, die die Hautkontaktfläche bildet (also insgesamt das aus der Tragfolie 328 und dem Basisteil gebildete Referenzelement) zusammen mit der Lanzette 330 bewegt, wobei der in Figur 29B dargestellte Nadelüberstand d die Stechtiefe definiert. Durch die Vorwärtsbewegung erfolgt der Einstich in einen in Figur 30A dargestellten Finger 342.

Im Rahmen der praktischen Erprobung dieser Ausführungsform wurde festgestellt, daß bei Verwendung sehr dünner Folien (mit den oben angegebenen bevorzugten Dicken) überraschenderweise sehr gut reproduzierbare Stechtiefen resultieren, selbst wenn die Tragfolie 328 oder Reste der Deckfolie 329 im Bereich der Hautkontaktfläche 11 keinen glatten Überzug bilden, sondern beispielsweise Falten werfen. Deswegen können bei dieser Ausführungsform der Erfindung deren Vorteile in idealer Weise mit den Vorteilen eines Nadelelementbandes kombiniert werden. Dies gilt insbesondere, wenn das Band nicht nur Nadelelemente (Lanzetten), sondern alternierend auch Testelemente trägt und das System als integriertes System ausgebildet ist, dem nicht nur die Blutentnahme, sondern auch die Analyse durchgeführt wird. Ein kombiniertes Analyseelement-Band ermöglichst auf einfache Weise die Realisierung der bei solchen integrierten Systemen notwendigen Transportfunktionen, während gleichzeitig hygienische Probleme durch die Verwendung des Trägerbandes als Bestandteil des Referenzelementes im Bereich der Hautkontaktfläche vermieden werden. Dabei werden die Testelemente wegen der Empfindlichkeit der darin enthaltenen Reagenzien zweckmäßigerweise gesondert hergestellt und durch Kleben auf der Tragfolie 328 befestigt.

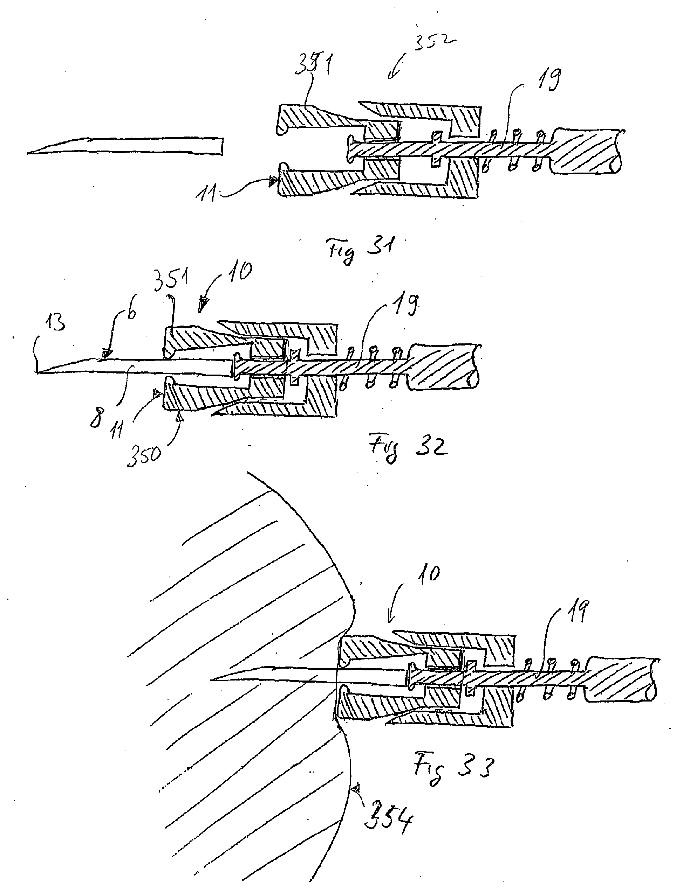

In den Figuren 31 bis 33 sind die für die Funktion wesentlichsten Teile eines Stechsystems dargestellt, bei dem der Kopplungsmechanismus 3, durch den ein Nadelelement 6 und ein Referenzelement 10 über eine Verbindungsstange 19 mit einem nicht dargestellten Antrieb verbunden sind, besonders einfach und platzsparend ausgebildet ist. Das Nadelelement 6 besteht im dargestellten Fall nur aus einer Metallnadel 8. Für den dargestellten Kopplungsmechanismus ist charakteristisch, daß das Stechtiefenreferenzelement 10 gleichzeitig einen Greifer 350 bildet, der das Nadelelement 6 zangenartig umgreift und dadurch fixiert. Im dargestellten Fall werden Greifarme 351 des Greifers 350 durch eine Schließmechanik 352 betätigt, die nicht näher erläutert werden muß, weil geeignete mechanische Prinzipien (beispielsweise zum Halten von Minen in Druckbleistiften) bekannt sind. Entscheidend ist, daß der Greifer so ausgebildet ist, daß er im geöffneten Zustand (Figur 31) über das Nadelelement 6 geschoben werden kann und die Greifarme 351 anschließend geschlossen werden können, wobei sie das Nadelelement 8 durch seitlichen Druck in Richtung auf dessen Zentrum fixieren (Figur 32). Im einfachsten Fall wird dabei die beim Einstich an der Hautoberfläche 354 anliegenden Hautkontaktfläche 11 des Stechtiefenreferenzelementes 10 (Figur 33) von einer entsprechend geformten vorderen Stirnfläche des Greifers 350 gebildet.

Bei der in den Figuren 31 bis 33 dargestellten Ausführungsform dient das Stechtiefenreferenzelement 10 zugleich als Halterung für das Nadelelement. Der Greifer 350 bildet Fixierungsmittel, mit denen das Nadelelement 6 zur Einstellung der Stechtiefe in unterschiedlichen Longitudinalpositionen relativ zu dem Stechtiefenreferenzelement 10 fixiert werden kann. Im dargestellten Fall erfolgt die Einstellung der Stechtiefe durch Drehen der Verbindungsstange 19 relativ zu den Greifarmen 351. Es sind jedoch andere Realisierungen des beschriebenen Konstruktionsprinzips, auch hinsichtlich der Fixierungsmittel, möglich.

In diesem Fall gehört die Hautkontaktfläche zu den wiederverwendbaren Teilen des Stechgeräts. Um Verschmutzungen zu vermeiden und die hygienischen Verhältnisse zu verbessern, kann es vorteilhaft sein, am vorderen Ende des Greifers einen einmal verwendbaren Schutzüberzug, beispielsweise in Form einer Folie oder in Form von auswechselbaren Kappen aus Kunststoff vorzusehen. In diesem Fall bildet der Greifer 350 nicht unmittelbar die Hautkontaktfläche, sondern ein Referenzelementbasisteil, dessen Oberfläche (ähnlich wie bei den Figuren 26 bis 30) von dem Überzug bedeckt ist, der die eigentliche Hautkontaktfläche bildet.

Die dargestellten Beispiele zeigen, daß zahlreiche Varianten der Erfindung möglich sind. Dies bezieht sich beispielsweise auf die Kupplungen, die voneinander getrennte Verbindungen des Nadelelementes und des Stechtiefenreferenzelementes mit den entsprechenden Positionierungsteilen des Kopplungsmechanismus herstellen. Allen Ausführungsformen ist jedoch gemeinsam, daß mindestens das Nadelelement, vorzugsweise auch das Referenzelement eine Kupplungsstruktur aufweist, die mit einem korrespondierenden Kupplungsprofil des entsprechenden Positionierungsteils zusammenwirkt. Die Begriffe "Kupplungsstruktur" bzw. "Kupplungsprofil" bezeichnen dabei ganz allgemein jede Gestaltung der genannten Elemente, durch die eine mindestens unidirektionale, vorzugsweise bidirektionale Kopplung in dem erläuterten Sinne bewirkt wird.

Auch hinsichtlich des Zeitpunktes, zu dem die Kopplung erfolgt, sind zahlreiche Varianten möglich. Insbesondere können für manche Anwendungszwecke Konstruktionen vorteilhaft sein, bei denen die Kopplung durch die Nadelelementkupplung und/oder die Referenzelementkupplung erst während der Vortriebsphase der Stechbewegung erfolgt. Auch Ausführungsformen, bei denen diese Kupplungen während der Rückzugsphase der Stechbewegung vor Erreichen der Ausgangsposition des Stechantriebs getrennt werden, sind für manche Anwendungszwecke vorteilhaft.

Selbstverständlich können statt der in den Figuren dargestellten Schraubenfedern auch andere Federeinrichtungen verwendet werden, wie beispielsweise Blattfedern, Tellerfedern oder sich gegenseitig abstoßende Magnete. Der Antrieb während der Rückzugsphase erfolgt vorzugsweise mit gesonderten Federelementen der gleichen oder einer anderen Ausführungsform.

Wie eingangs erläutert, eignet sich die Erfindung insbesondere für integrierte Systeme, bei denen die Funktionen der Blutgewinnung und der Analyse in einem Gerät vereinigt sind. Im Falle eines Mikrosamplers erfolgt diese Integration vorzugsweise dadurch, dass in dessen Probenaufnahmebereich die für die Analyse erforderlichen Reagenzien und sonstigen Komponenten vorhanden sind. Insoweit unterscheiden sich für die Erfindung geeignete Mikrosampler nicht von bekannten Systemen. Auch die Stecheinheiten ohne Kapillarkanal können in Verbindung mit der Erfindung in vorteilhafter Weise in integrierten Systemen verwendet werden, wobei in diesem Fall die Stecheinheit nach dem Stechvorgang rasch zurückgezogen wird, so dass die aus der Haut austretende Probenflüssigkeit in einen Kapillarkanal eines Analyseelementes eindringen kann, das zu diesem Zweck innerhalb des Stechgerätes in Kontakt mit der aus der Haut austretenden Probenflüssigkeit gebracht wird.

## Patentansprüche

1. Stechsystem zur Entnahme einer Körperflüssigkeit aus der Haut eines Menschen oder Tieres, mit
einem Nadelelement (6) zum Einstechen in die Haut,
einem Stechgerät (2), das einen Stechantrieb (4) einschließt, durch den eine Stechbewegung (24) des Nadelelementes (6), das mittels eines Kopplungsmechanismus (3) mit dem Stechantrieb (4) gekoppelt ist, angetrieben wird,
wobei
a) in einer Vortriebsphase der Stechbewegung (24) das Nadelelement (6) entlang einem vorbestimmten Einstichweg in einer Einstichrichtung bewegt wird, bis seine Spitze (13) in die Haut eindringt und in einer Rückzugsphase der Stechbewegung (24) das Nadelelement (6) nach Erreichen eines der Stechtiefe in die Haut entsprechenden Umkehrpunktes wieder zurückgezogen wird,
b) ein vorgegebener Wert der Stechtiefe mittels eines Stechtiefenreferenzelementes (10) gewährleistet wird, das eine Hautkontaktfläche (11) aufweist, wobei der vorgegebene Wert der Stechtiefe durch den Abstand in Einstichrichtung zwischen der Hautkontaktfläche (11) und der Position der Spitze (13) des Nadelelementes (6) an dem Umkehrpunkt der Stechbewegung bestimmt wird,
c) das Nadelelement (6) und das Stechtiefenreferenzelement (10) relativ zueinander in Einstichrichtung verschiebbare Elemente sind und die Stechtiefe dadurch einstellbar ist, dass der bei Erreichen des Umkehrpunktes vorhandene Abstand (d) zwischen der Hautkontaktfläche (11) des Stechtiefenreferenzelementes (10) und die Spitze (13) des Nadelelementes (6) verändert wird,
d) das Stechtiefenreferenzelement (10) durch den Kopplungsmechanismus (3) derartig mit dem Antrieb gekoppelt ist, dass es zumindest während eines Teils der Vortriebsphase der Stechbewegung gemeinsam mit dem Nadelelement (6) bewegt wird, und mindestens an dem Umkehrpunkt der Stechbewegung eine definierte Längsposition in Einstichrichtung relativ zu dem Nadelelement (6) hat, und
e) der Kopplungsmechanismus (3) eine Nadelelementkupplung (16) mit einem Nadelelementpositionierungsteil (16a) einschließt, das einen Anschlag (16b) aufweist, der mit einem korrespondieren Anschlag (6b) des Nadelelementes (6) so zusammenwirkt, dass die Längsposition des Nadelelementes (6) mindestens an dem Umkehrpunkt der Stechbewegung durch den Kontakt dieser Anschläge (16b,6b) bestimmt wird, der Kopplungsmechanismus (3) eine Referenzelementkupplung (17) mit einem Referenzelementpositionierungsteil (17a) einschließt, das einen Anschlag (17b) aufweist, der mit einem korrespondierenden Anschlag (10b) des Stechtiefenreferenzelementes (10) so zusammenwirkt, dass die Längsposition des Stechtiefenreferenzelementes (10) mindestens an dem Umkehrpunkt der Stechbewegung durch den Kontakt dieser Anschläge (17b,10b) bestimmt wird, und die Longitudinalposition, in der sich die Positionierungsteile (16a,17a) am Umkehrpunkt befinden, relativ zueinander zur Einstellung der Stechtiefe veränderbar ist.

2. Stechsystem nach Anspruch 1, **dadurch gekennzeichnet, dass** während der Vortriebsphase der Stechbewegung, zumindest zum Kontaktierungszeitpunkt zwischen der Hautkontaktfläche (11) und der Hautoberfläche, der Abstand zwischen der Hautkontaktfläche (11) und der Spitze (13) des Nadelelementes (6) kleiner als der vorgegebene Wert der Stechtiefe ist, so dass die Haut durch das Auftreffen der Hautkontaktfläche gestrafft wird, bevor die Stechbewegung des Nadelelementes den Umkehrpunkt erreicht.

3. Stechsystem nach Anspruch 1, **dadurch gekennzeichnet, dass** sich während der Vortriebsphase, zumindest zum Kontaktierungszeitpunkt, die Spitze (13) des Nadelelementes (6) in Einstichrichtung hinter der Hautkontaktfläche (11) befindet, so dass die Haut durch das Auftreffen der Hautkontaktfläche gestrafft wird, bevor der Einstich in die Haut erfolgt.

4. Stechsystem nach einem der vorhergehenden Ansprüche, **dadurch gekennzeichnet, dass** der Druck, mit dem die Hautkontaktfläche (11) des Stechtiefenreferenzelementes (10) während der Vortriebsphase der Stechbewegung gegen die Hautoberfläche drückt, so hoch ist, dass dadurch die Beeinträchtigung der Reproduzierbarkeit der Stechtiefe durch eine mit dem Einstechen des Nadelelementes in die Haut verbundene Hautdeformation reduziert wird.

5. Stechsystem nach einem der vorhergehenden Ansprüche, **dadurch gekennzeichnet, dass** der Stechantrieb (4) während mindestens eines Teils der Vortriebsphase mit dem Nadelelement (6) gekoppelt ist und das Referenzelement (10) über eine Mitnehmereinrichtung (103) während mindestens eines Teils desjenigen Teils der Vortriebsphase, in dem das Nadelelement (6) mit dem Stechantrieb (4) gekoppelt ist, mit dem Nadelelement (6) und dadurch mit dem Stechantrieb (4) gekoppelt ist.

6. Stechsystem nach Anspruch 5, **dadurch gekennzeichnet, dass** die Mitnehmereinrichtung (103) je einen mit dem Nadelelement (6) bzw. dem Stechtiefenreferenzelement (10) gekoppelten Stechtiefenbegrenzungsanschlag (101,125) aufweist, und die Stechtiefenbegrenzungsanschläge (101,125) am Umkehrpunkt der Stechbewegung derartig aneinander anliegen, dass ihr relativer Abstand in Längsrichtung die Längsposition der Nadelspitze (13) relativ zu der Hautkontaktfläche (11) des Stechtiefenreferenzelementes (10) und damit die Stechtiefe definiert.

7. Stechsystem nach einem der Ansprüche 5 oder 6, **dadurch gekennzeichnet, dass** das Stechtiefenreferenzelement (10) während des dem Kontaktierungszeitpunkt vorausgehenden Abschnitts der Vortriebsphase der Stechbewegung über die Mitnehmereinrichtung (103) elastisch mit dem Nadelelement (6) und dadurch mit dem Stechantrieb (4) gekoppelt ist.

8. Stechsystem nach einem der Ansprüche 5 bis 7, **dadurch gekennzeichnet, dass** das Stechtiefenreferenzelement (10) über eine bidirektional wirkende Mitnehmereinrichtung (103) mit dem Nadelelement (6) und dadurch mit dem Stechantrieb (4) gekoppelt ist, wobei die Mitnehmereinrichtung (103) je einen mit dem Nadelelement (6) bzw. dem Stechtiefenreferenzelement (10) gekoppelten in der Rückzugsphase wirkenden Anschlag (143,144) aufweist.

9. Stechsystem nach Anspruch 8, **dadurch gekennzeichnet, dass** die Anschläge (143, 144) während mindestens eines Teils der Rückzugsphase derartig aneinander anliegen, dass dadurch die Position der Nadelspitze (13) relativ zu der Hautkontaktfläche (11) des Stechtiefenreferenzelement (10) definiert ist und die in der Rückzugsphase wirkenden Anschläge (143,144) derart positioniert sind, dass sie eine Reststechtiefe definieren, um die die Nadelspitze (13) gegenüber der Hautkontaktfläche (11) hervorsteht.

10. Stechsystem nach einem der vorhergehenden Ansprüche, **dadurch gekennzeichnet, dass** der Durchmesser der Hautkontaktfläche mindestens 1,5 mm und höchstens 6 mm beträgt.

11. Stechsystem nach einem der vorhergehenden Ansprüche, **dadurch gekennzeichnet, dass** das Stechtiefenreferenzelement (10) während eines Teils der Vortriebsphase mittels eines Referenzbasisteils (146) auf einem Referenzelementlager (105) in einer Ruheposition ruht, und ausgehend von der Ruheposition in der Vortriebsphase von dem Stechantrieb (4) in Einstichrichtung beschleunigt wird.

12. Stechsystem nach Anspruch 11, **dadurch gekennzeichnet, dass** der Teilabschnitt der Stechbewegung, während dessen das Stechtiefenreferenzelement (10) gemeinsam mit dem Nadelelement (6) bewegt wird, maximal 100 msec, bevorzugt maximal 50 msec und besonders bevorzugt maximal 10 msec dauert.

13. Stechsystem nach einem der vorhergehenden Ansprüche, **dadurch gekennzeichnet, dass** das Stechgerät (2) mehrfach verwendbar ist und eine Halterung (27) einschließt, mittels der jeweils eine Stecheinheit (1) auswechselbar mit dem Stechantrieb (4) gekoppelt werden kann, wobei mindestens die Hautkontaktfläche (11) des Stechtiefenreferenzelements (10) an einem zur einmaligen Verwendung vorgesehenen disposiblen Element ausgebildet ist.

14. Stechsystem nach Anspruch 13, **dadurch gekennzeichnet, dass** das Stechtiefenreferenzelement (10) einen Referenzelementkörper (12) aufweist, der sich von der Hautkontaktfläche (11) entgegen der Einstichrichtung erstreckt und einen Körperteil (7) des Nadelelementes (6) zumindest teilweise umgreift und die Längsposition des Nadelelementes (6) relativ zu der Längsposition des Referenzelementkörpers (12) während der Rückzugsphase der Stechbewegung oder danach vor dem Entfernen der Stecheinheit (1) aus der Halterung (27) derartig verändert wird, dass die Nadelspitze (13) zum Schutz vor Verletzungen hinter die Hautkontaktfläche (11) des Stechtiefenreferenzelementes (10) zurückgezogen wird.

15. Stechsystem nach einem der Ansprüche 13 oder 14, **dadurch gekennzeichnet, dass** es ein Magazin mit einer Mehrzahl von Nadelelementen (6) einschließt.

16. Stechsystem nach einem der Ansprüche 13 bis 15, **dadurch gekennzeichnet, dass** es ein Stechtiefenreferenzelement (10) aufweist, das so ausgebildet und angeordnet ist, dass es mit mehreren Nadelelementen (6) des Magazins (85) zusammenwirken kann, die nacheinander in eine Position transportiert werden, in der jeweils ein Nadelelement (6) mindestens während eines Teils der Stechbewegung gemeinsam mit dem Stechtiefenreferenzelement (10) bewegt werden kann.

17. Stechsystem nach einem der Ansprüche 13 bis 16, **dadurch gekennzeichnet, dass**
es ein Nadelelementband (331) mit einer Mehrzahl von Nadelelementen (6) einschließt, die auf einer Tragfolie (328) derartig fixiert und eingesiegelt sind, dass ihre Nadelspitzen (13) hygienisch geschützt sind,
es eine Transporteinrichtung aufweist, durch die das Nadelelementband (331) in dem Stechgerät derartig schrittweise transportiert wird, dass sich jeweils ein Nadelelement (6) in einer Stechstation (332) bei einem Referenzelementbasisteil (333) befindet,
es eine Stechvorbereitungseinrichtung (335) aufweist, durch die das in der Stechstation (332) befindliche Nadelelement (6) in eine Stechposition gebracht wird, bei der seine Nadelspitze (13) freigelegt und in Einstichrichtung orientiert ist, während die Tragfolie das Referenzelementbasisteil (333) in der Umgebung der Nadelspitze (13) abdeckt und die Hautkontaktfläche (11) bildet,
wobei das Referenzelementbasisteil (333) während der Stechbewegung gemeinsam mit der die Hautkontaktfläche (11) bildenden Tragfolie (328) bewegt wird und das Stechtiefenreferenzelement (10) bildet.

18. Stechsystem nach Anspruch 17, **dadurch gekennzeichnet, dass** die Tragfolie höchstens 100 µm, vorzugsweise höchstens 50 µm und besonders bevorzugt höchstens 30 µm dick ist.

19. Stechsystem nach Anspruch 17 oder 18, **dadurch gekennzeichnet, dass** die Nadelelemente mittels einer Deckfolie (329) eingesiegelt sind, deren Dicke höchstens 50 µm, vorzugsweise höchstens 25 µm und besonders bevorzugt höchstens 15 µm beträgt.

20. Stechsystem nach einem der Ansprüche 17 bis 19, **dadurch gekennzeichnet, dass** das Stechtiefenreferenzelement (10) als Halterung für das Nadelelement (6) dient und Fixierungsmittel aufweist, mittels denen das Nadelelement (6) zur Einstellung der Stechtiefe in unterschiedlichen Longitudinalpositionen relativ zu dem Stechtiefenreferenzelement (10) fixiert werden kann.

21. Disposible Stecheinheit (1) zur Verwendung mit einem Stechgerät (2) bei dem jeweils eine disposible Stecheinheit (1) auswechselbar mit einem in dem Stechgerät (2) vorgesehenen Stechantrieb (4) koppelbar ist, mit
- einem Nadelelement (6) zum Einstechen in die Haut und
- einem Stechtiefenreferenzelement (10), durch das ein vorgegebener Wert der Stechtiefe gewährleistet wird,
wobei
a) das Nadelelement (6) und das Stechtiefenreferenzelement (10) getrennte, relativ zueinander in Einstichrichtung verschiebbare Elemente der Stecheinheit (1) sind
b) das Nadelelement (6) und das Stechtiefenreferenzelement (10) durch einen Kopplungsmechanismus (3) derartig mit dem Antrieb des Stechgerätes koppelbar sind, dass das Stechriefenreferenzelement zumindest während eines Teils der Vortriebsphase der Stechbewegung gemeinsam mit dem Nadelelement (6) bewegt wird, und mindestens an dem Umkehrpunkt der Stechbewegung eine definierte Längsposition in Einstichrichtung relativ zu dem Nadelelement (6) hat,
c) die Längsposition in Einstichrichtung (24) des Stechtiefenreferenzelementes (10) relativ zu dem Nadelelement (6) und damit der Abstand zwischen der Hautkontaktfläche (11) und der Nadelspitze (13) zur Einstellung der vorgegebenen Stechtiefe veränderbar ist, wobei die Stechtiefe dadurch einstellbar ist, dass der bei Erreichen des Umkehrpunktes vorhandene Abstand (d) zwischen der Hautkontaktfläche (11) des Stechtiefenreferenzelementes (10) und die Spitze (13) des Nadelelementes (6) verändert wird, und
d) das Nadelelement (6) einen Anschlag (6b) aufweist, der mit einem korrespondierenden Anschlag (16b) eines Nadelelementpositionierungsteils (16a) des Kopplungsmechanismus (3) so zusammenwirkt, dass die Längsposition des Nadelelementes (6) mindestens an dem Umkehrpunkt der Stechbewegung durch den Kontakt dieser Anschläge (16b,6b) bestimmt wird,
e) das Stechtiefenreferenzelement (10) einen Anschlag (10b) aufweist, der mit einem korrespondierenden Anschlag (17b) eines Referenzelementpositionierungsteils (17a) des Kopplungsmechanismus (3) so zusammenwirkt, dass die Längsposition des Stechtiefenreferenzelementes (10) mindestens an dem Umkehrpunkt der Stechbewegung durch den Kontakt dieser Anschläge (17b,10b) bestimmt wird.

22. Disposible Stecheinheit nach Anspruch 21, **dadurch gekennzeichnet, dass** das Nadelelement (6) eine Kupplungsstruktur aufweist, die mit einem korrespondierenden Kupplungsprofil des Nadelelementpositionierungsteils (16a) zusammenwirkt, um das Nadelelement (6) mit dem Nadelelementpositionierungsteil (16a) zu koppeln.

23. Disposible Stecheinheit nach Anspruch 22, **dadurch gekennzeichnet, dass** auch das Stechtiefenreferenzelement (10) eine Kupplungsstruktur aufweist, die mit einem korrespondierenden Kupplungsprofil des Referenzelementpositionierungsteils zusammenwirkt, um das Referenzelement mit dem Referenzelementpositionierungsteil zu koppeln.

## Claims

1. Puncturing system for withdrawing a body fluid from the skin of a human or animal, comprising
a needle element (6) for piercing into the skin,
a puncturing instrument (2), which includes a puncturing drive (4), by which a puncturing movement (24) of the needle element (6), which is coupled to the puncturing drive (4) by means of a coupling mechanism (3), is driven,
wherein
a) in a forward phase of the puncturing movement (24), the needle element (6) is moved along a predetermined movement path in a piercing direction until its tip (13) penetrates into the skin and, in a retraction phase of the puncturing movement (24), the needle element (6) is retracted after reaching a reversal point corresponding to the puncturing depth in the skin,
b) a predefined value of the puncturing depth is ensured by means of a puncturing depth reference element (10), which has a skin contact area (11), the predefined value of the puncturing depth being determined by the distance in the piercing direction between the skin contact area (11) and the position of the tip (13) of the needle element (6) at the reversal point of the puncturing movement,
c) the needle element (6) and the puncturing depth reference element (10) are separate elements which can be shifted relative to each other in the piercing direction, and the puncturing depth is adjustable by changing the distance (d) existing at the reversal point between the skin contact area (11) of the puncturing depth reference element (10) and the tip (13) of the needle element (6),
d) the puncturing depth reference element (10) is coupled to the drive by the coupling mechanism (3) in such a manner that it is moved together with the needle element (6) at least during a part of the forward phase of the puncturing movement, and has a defined longitudinal position in the piercing direction in relation to the needle element (6) at least at the reversal point of the puncturing movement,
e) the coupling mechanism (3) includes a needle element coupler (16) comprising a needle element positioning part (16a), which has a stop (16b), cooperating with a corresponding stop (6b) of the needle element (6) in such a manner that the longitudinal position of the needle element (6) is at least at the reversal point of the puncturing movement determined by the contact of said stops (16b, 6b), the coupling mechanism (3) includes a reference element coupler (17) comprising a reference element positioning part (17a), which has a stop (17b), cooperating with a corresponding stop (10b) of the puncturing depth reference element (10) in such a manner that the longitudinal position of the puncturing depth reference element (10) is determined, at least at the reversal point of the puncturing movement, by the contact of said stops (17b, 10b), and the longitudinal position at which the positioning parts (16a, 17a) are located at the reversal point in relation to one another is changeable for adjusting the puncturing depth.

2. Puncturing system according to claim 1, **characterized in that**, during the forward phase of the puncturing movement, at least at the contact instant at which the skin contact area (11) contacts the skin surface, the distance between the skin contact area (11) and the tip (13) of the needle element (6) is less than the predefined value of the puncturing depth, so that the skin is tensioned by the impact of the skin contact area, before the puncturing movement of the needle element reaches the reversal point.

3. Puncturing system according to claim 1, **characterized in that**, during the forward phase, at least at the contact instant, the tip (13) of the needle element (6) is located behind the skin contact area (11) in the piercing direction, so that the skin is tensioned by the impact of the skin contact area before the piercing into the skin occurs.

4. Puncturing system according to any one of the preceding claims, **characterized in that** the pressure with which the skin contact area (11) of the puncturing depth reference element (10) presses against the skin surface during the forward phase of the puncturing movement is so high that deterioration of the reproducibility of the puncturing depth by a skin deformation, occurring during piercing of the needle element into the skin, is reduced thereby.

5. Puncturing system according to any one of the preceding claims, **characterized in that** the puncturing drive (4) is coupled to the needle element (6) during at least a part of the forward phase and the reference element (10) is coupled to the needle element (6) and thus to the puncturing drive (4) by means of a co-transport device (103) during at least a part of that part of the forward phase in which the needle element (6) is coupled to the puncturing drive (4).

6. Puncturing system according to claim 5, **characterized in that** the co-transport device (103) has puncturing depth limit stops (101, 125), one of which is coupled to each of the needle element (6) and the puncturing depth reference element (10), and the puncturing depth limit stops (101, 125) are in engagement with one another at the reversal point of the puncturing movement in such a manner that their relative distance in the longitudinal direction defines the longitudinal position of the needle tip (13) in relation to the skin contact area (11) of the puncturing depth reference element (10) and thus the puncturing depth.

7. Puncturing system according to any one of the claims 5 or 6, **characterized in that** the puncturing depth reference element (10) is, during the section of the forward phase of the puncturing movement preceding the contact instant, elastically coupled via the co-transport device (103) to the needle element (6) and thus to the puncturing drive (4).

8. Puncturing system according to any one of the claims 5 to 7, **characterized in that** the puncturing depth reference element (10) is coupled via a bidirectionally acting co-transport device (103) to the needle element (6) and thus to the puncturing drive (4), wherein the co-transport device (103) comprises stops (143, 144) acting in the retraction phase, one of said stops being coupled to each of the needle element (6) and the puncturing depth reference element (10).

9. Puncturing system according to claim 8, **characterized in that** the stops (143, 144) are in engagement with one another during at least a part of the retraction phase in such a manner that the position of the needle tip (13) in relation to the skin contact area (11) of the puncturing depth reference element (10) is defined thereby and the stops (143, 144) acting in the retraction phase are positioned in such a manner that they define a residual puncturing depth by which the needle tip (13) protrudes in relation to the skin contact area (11).

10. Puncturing system according to any one of the preceding claims, **characterized in that** the diameter of the skin contact area is at least 1.5 mm and at most 6 mm.

11. Puncturing system according to any one of the preceding claims, **characterized in that** the puncturing depth reference element (10) rests, during a part of the forward phase, in a rest position by means of a reference base part (146) on a reference element bearing (105), and the puncturing depth reference element in the forward phase, starting from the rest position, is accelerated in the piercing direction by the piercing drive (4).

12. Puncturing system according to claim 11, **characterized in that** the partial section of the puncturing movement, during which the puncturing depth reference element (10) is moved together with the needle element (6), lasts at most 100 ms, preferably at most 50 ms and particularly preferably at most 10 ms.

13. Puncturing system according to any one of the preceding claims, **characterized in that** the puncturing instrument (2) is a multiple-use item and comprises a holder (27), by means of which a puncturing unit (1) each may be coupled interchangeably to the puncturing drive (4), wherein at least the skin contact area (11) of the puncturing depth reference element (10) is formed on a single-use disposable element.

14. Puncturing system according to claim 13, **characterized in that** the puncturing depth reference element (10) has a reference element body (12), which extends from the skin contact area (11) opposite to the piercing direction and at least partially encloses a body part (7) of the needle element (6) and **in that** the longitudinal position of the needle element (6) in relation to the longitudinal position of the reference element body (12) is changed during the retraction phase of the puncturing movement or after-wards before the removal of the puncturing unit (1) from the holder (27) in such a manner that the needle tip (13) is retracted behind the skin contact area (11) of the puncturing depth reference element (10) for protection from injuries.

15. Puncturing system according to any one of the claims 13 or 14, **characterized in that** it includes a magazine including a plurality of needle elements (6).

16. Puncturing system according to any one of the claims 13 to 15, **characterized in that** it has a puncturing depth reference element (10), which is adapted and arranged for cooperation with a plurality of needle elements (6) of the magazine (85), which are transported in sequence into a position in which one needle element (6) at a time may be moved together with the puncturing depth reference element (10) during at least a part of the puncturing movement.

17. Puncturing system according to any one of the claims 13 to 16, **characterized in that**
it includes a needle element strip (331) including a plurality of needle elements (6), which are fixed on a carrier film (328) and sealed in such a manner that their needle tips (13) are hygienically protected it has a transport device, by which the needle element strip (331) is transported step-by-step in the puncturing instrument such that one needle element (6) at a time is located in a puncture station (332) at a reference element base part (333),
it comprises a puncture preparation device (335), by which the needle element (6) located in the puncture station (332) is brought into a puncture position, in which its needle tip (13) is exposed and oriented in the piercing direction, while the carrier film covers the reference element base part (333) in the surroundings of the needle tip (13) and forms the skin contact area (11),
the reference element base part (333) is moved together with the carrier film (328) forming the skin contact area (11) during the puncturing movement and the reference element base part (333) forms the puncturing depth reference element (10).

18. Puncturing system according to claim 17, **characterized in that** the carrier film is at most 100 µm thick, preferably at most 50 µm thick, particularly preferably at most 30 µm thick.

19. Puncturing system according to claim 17 or 18, **characterized in that** the needle elements are sealed by means of a cover film (329), having a thickness of at most 60 µm, preferably of at most 25 µm, particularly preferably of at most 15 µm.

20. Puncturing system according to any one of the claims 17 to 19, **characterized in that** the puncturing depth reference element (10) is adapted to function as a holder for the needle element (6) and has fixing means, by means of which the needle element (6) may be fixed in different longitudinal positions in relation to the puncturing depth reference element (10) for adjustment of the puncturing depth.

21. Disposable puncturing unit (1) for use in a puncturing instrument (2), in which one disposable puncturing unit (1) at a time may be replaceably coupled to a puncturing drive (4) provided in the puncturing instrument (2), comprising
- a needle element (6) for piercing into the skin, and
- a puncturing depth reference element (10), by which a predefined value of the puncturing depth is ensured,
wherein
a) the needle element (6) and the puncturing depth reference element (10) are separate elements of the puncturing unit (1), which can be shifted relative to each other in the piercing direction,
b) the needle element (6) and the puncturing depth reference element (10) may be coupled to the drive of the puncturing instrument by a coupling mechanism (3) in such a manner that the puncturing depth reference element (10) is moved together with the needle element (6) at least during a part of the forward phase of the puncturing movement and has a defined longitudinal position in the piercing direction in relation to the needle element (6) at least at the reversal point of the puncturing movement,
c) the longitudinal position in the piercing direction (24) of the puncturing depth reference element (10) in relation to the needle element (6) and thus the distance between the skin contact area (11) and the needle tip (13) is changeable to adjust the predefined puncturing depth, wherein the puncturing depth is adjustable by changing the distance (d) between the skin contact area (11) of the puncturing depth reference element (10) and the tip (13) of the needle element (6) existing at the reversal point, and
d) the needle element (6) has a stop (6b) cooperating with a corresponding stop (16b) of a needle element positioning part (16a) of the coupling mechanism (3) such that the longitudinal position of the needle element (6) is at least at the reversal point of the puncturing movement determined by the contact of said stops (16b, 6b),
e) the puncturing depth reference element (10) has a stop (10b) cooperating with a corresponding stop (17b) of a reference element positioning part (17a) of the coupling mechanism (3) in such a manner that the longitudinal position of the puncturing depth reference element (10) is determined at least at the reversal point of the puncturing movement by the contact of said stops (17b, 10b).

22. Disposable puncturing unit according to claim 21, **characterized in that** the needle element (6) has a coupling structure, which cooperates with a corresponding coupling profile of the needle element positioning part (16a) for coupling the needle element (6) to the needle element positioning part (16a).

23. Disposable puncturing unit according to claim 22, **characterized in that** the puncturing depth reference element (10) also has a coupling structure, which cooperates with a corresponding coupling profile of the reference element positioning part for coupling the reference element to the reference element positioning part.

## Revendications

1. Système de piqûre destiné au prélèvement d'un liquide organique à travers la peau d'un être humain ou d'un animal, comprenant
un élément aiguille (6) pour piquer la peau,
un appareil de piqûre (2) incluant un entraînement de piqûre (4) par lequel est commandé un mouvement de piqûre (24) de l'élément aiguille (6) couplé à l'entraînement de piqûre (4) au moyen d'un mécanisme de couplage (3),
a) pendant une phase d'avancement du mouvement de piqûre (24), l'élément aiguille (6) étant déplacé dans un sens de piqûre sur un trajet de piqûre prédéterminé jusqu'à ce que sa pointe (13) pénètre dans la peau, et pendant une phase de retour du mouvement de piqûre (24), l'élément aiguille (6) étant de nouveau retiré après avoir atteint un point d'inversion correspondant à la profondeur de piqûre dans la peau,
b) une valeur prédéterminée de la profondeur de piqûre étant garantie au moyen d'un élément de référence de profondeur de piqûre (10) qui présente une surface de contact avec la peau (11), la valeur prédéterminée de la profondeur de piqûre étant déterminée par la distance dans le sens de piqûre entre la surface de contact avec la peau (11) et la position de la pointe (13) de l'élément aiguille (6) au point d'inversion du mouvement de piqûre,
c) l'élément aiguille (6) et l'élément de référence de profondeur de piqûre (10) étant des éléments déplaçables l'un par rapport à l'autre dans le sens de piqûre, et la profondeur de piqûre étant réglable en modifiant la distance (d) qui existe entre la surface de contact avec la peau (11) de l'élément de référence de profondeur de piqûre (10) et la pointe (13) de l'élément aiguille (6) lorsque le point d'inversion est atteint,
d) l'élément de référence de profondeur de piqûre (10) étant couplé à l'entraînement par le mécanisme de couplage (3), de sorte que, au moins pendant une partie de la phase d'avancement du mouvement de piqûre, il est déplacé conjointement avec l'élément aiguille (6) et a, au moins au point d'inversion du mouvement de piqûre, une position longitudinale définie dans le sens de piqûre par rapport à l'élément aiguille (6), et
e) le mécanisme de couplage (3) incluant un coupleur (16) de l'élément aiguille doté d'un élément de positionnement (16a) de l'élément aiguille présentant une butée (16b) qui coopère avec une butée correspondante (6b) de l'élément aiguille (6), de sorte que la position longitudinale de l'élément aiguille (6) au moins au point d'inversion du mouvement de piqûre est déterminée par le contact de ces butées (16b, 6b), le mécanisme de couplage (3) incluant un coupleur (17) de l'élément de référence doté d'un élément de positionnement (17a) de l'élément de référence présentant une butée (17b) qui coopère avec une butée correspondante (10b) de l'élément de référence de profondeur de piqûre (10), de sorte que la position longitudinale de l'élément de référence de profondeur de piqûre (10) au moins au point d'inversion du mouvement de piqûre est déterminée par le contact de ces butées (17b, 10b), et la position longitudinale dans laquelle se trouvent les éléments de positionnement (16a, 17a) au point d'inversion étant modifiable l'une par rapport à l'autre pour régler la profondeur de piqûre.

2. Système de piqûre selon la revendication 1, **caractérisé en ce que** pendant la phase d'avancement du mouvement de piqûre, au moins au moment du contact entre la surface de contact avec la peau (11) et la surface de la peau, la distance entre la surface de contact avec la peau (11) et la pointe (13) de l'élément aiguille (6) est inférieure à la valeur prédéterminée de la profondeur de piqûre, de sorte que la peau est tendue par l'impact de la surface de contact avec la peau avant que le mouvement de piqûre de l'élément aiguille n'atteigne le point d'inversion.

3. Système de piqûre selon la revendication 1, **caractérisé en ce que** pendant la phase d'avancement, au moins au moment du contact, la pointe (13) de l'élément aiguille (6) se trouve dans le sens de piqûre derrière la surface de contact avec la peau (11), de sorte que la peau est tendue par l'impact de la surface de contact avec la peau avant que la piqûre de la peau n'ait lieu.

4. Système de piqûre selon l'une des revendications précédentes, **caractérisé en ce que** pression avec laquelle la surface de contact avec la peau (11) de l'élément de référence de profondeur de piqûre (10) est appliquée contre la surface de la peau pendant la phase d'avancement du mouvement de piqûre est si élevée que l'altération de la reproductibilité de la profondeur de piqûre par une déformation de la peau liée à l'introduction de l'élément aiguille dans la peau s'en voit réduite.

5. Système de piqûre selon l'une des revendications précédentes, **caractérisé en ce que** l'entraînement de piqûre (4) est couplé pendant au moins une partie de la phase d'avancement à l'élément aiguille (6) et l'élément de référence (10), par l'intermédiaire d'un dispositif entraîneur (103), est couplé pendant au moins une partie de ladite partie de la phase d'avancement dans laquelle l'élément aiguille (6) est couplé à l'entraînement de piqûre (4), à l'élément aiguille (6) et donc à l'entraînement de piqûre (4).

6. Système de piqûre selon la revendication 5, **caractérisé en ce que** le dispositif entraîneur (103) présente des butées de limitation de profondeur de piqûre (101, 125) couplés respectivement à l'élément aiguille (6) et à l'élément de référence de profondeur de piqûre (10), et les butées de limitation de profondeur de piqûre (101, 125) sont appliquées l'une contre l'autre au point d'inversion du mouvement de piqûre, de sorte que leur distance relative dans le sens longitudinal définit la position longitudinale de la pointe d'aiguille (13) par rapport à la surface de contact avec la peau (11) de l'élément de référence de profondeur de piqûre (10) et donc la profondeur de piqûre.

7. Système de piqûre selon l'une des revendications 5 ou 6, **caractérisé en ce que** pendant la partie de la phase d'avancement du mouvement de piqûre précédant le moment de contact, l'élément de référence de profondeur de piqûre (10) est couplé élastiquement par l'intermédiaire du dispositif entraîneur (103) à l'élément aiguille (6) et donc à l'entraînement de piqûre (4).

8. Système de piqûre selon l'une des revendications 5 à 7, **caractérisé en ce que** l'élément de référence de profondeur de piqûre (10) est couplé par l'intermédiaire d'un dispositif entraîneur à effet bidirectionnel (103) à l'élément aiguille (6) et donc à l'entraînement de piqûre (4), ledit dispositif entraîneur (103) présentant des butées (143, 144) couplées respectivement à l'élément aiguille (6) et à l'élément de référence de profondeur de piqûre (10) et agissant pendant la phase de retour.

9. Système de piqûre selon la revendication 8, **caractérisé en ce que** pendant au moins une partie de la phase de retour, les butées (143, 144) sont appliquées l'une contre l'autre de telle sorte que cela définit la position de la pointe d'aiguille (13) par rapport à la surface de contact avec la peau (11) de l'élément de référence de profondeur de piqûre (10) et les butées (143, 144) agissant pendant la phase de retour sont positionnées de telle sorte qu'elles définissent une profondeur de piqûre restante correspondant au dépassement de la pointe d'aiguille (13) par rapport à la surface de contact avec la peau (11).

10. Système de piqûre selon l'une des revendications précédentes, **caractérisé en ce que** le diamètre de la surface de contact avec la peau est au minimum de 1,5 mm et au maximum de 6 mm.

11. Système de piqûre selon l'une des revendications précédentes, **caractérisé en ce que** l'élément de référence de profondeur de piqûre (10) repose pendant une partie de la phase d'avancement au moyen d'un élément de base de référence (146) sur un appui (105) de l'élément de référence dans une position de repos et est propulsé à partir de la position de repos pendant la phase d'avancement par l'entraînement de piqûre (4) dans le sens de piqûre.

12. Système de piqûre selon la revendication 11, **caractérisé en ce que** la partie du mouvement de piqûre pendant laquelle l'élément de référence de profondeur de piqûre (10) est déplacé conjointement avec l'élément aiguille (6), dure au maximum 100 msec, de préférence au maximum 50 msec et mieux encore au maximum 10 msec.

13. Système de piqûre selon l'une des revendications précédentes, **caractérisé en ce que** l'appareil de piqûre (2) est utilisable plusieurs fois et inclut un support (27) au moyen duquel chaque fois un ensemble de piqûre (1) peut être couplé de manière interchangeable à l'entraînement de piqûre (4), au moins la surface de contact avec la peau (11) de l'élément de référence de profondeur de piqûre (10) étant réalisée sur un élément jetable prévu pour un usage unique.

14. Système de piqûre selon la revendication 13, **caractérisé en ce que** l'élément de référence de profondeur de piqûre (10) présente un corps (12) qui s'étend de la surface de contact avec la peau (11) dans le sens contraire au sens de piqûre et entoure au moins partiellement une partie du corps (7) de l'élément aiguille (6) et **en ce que** la position longitudinale de l'élément aiguille (6) par rapport à la position longitudinale du corps (12) de l'élément de référence est modifiée pendant la phase de retour du mouvement de piqûre ou après avant de retirer l'ensemble de piqûre (1) du support (27), de telle sorte que la pointe d'aiguille (13) est repositionnée derrière la surface de contact avec la peau (11) de l'élément de référence de profondeur de piqûre (10) pour éviter toute blessure.

15. Système de piqûre selon l'une des revendications 13 ou 14, **caractérisé en ce qu'**il inclut un magasin comprenant une pluralité d'éléments aiguilles (6).

16. Système de piqûre selon l'une des revendications 13 à 15, **caractérisé en ce qu'**il présente un élément de référence de profondeur de piqûre (10), adapté et disposé de façon à pouvoir coopérer avec plusieurs éléments aiguilles (6) du magasin (85) transportés l'un après l'autre dans une position dans laquelle chaque fois un élément aiguille (6) peut être déplacé conjointement avec l'élément de référence de profondeur de piqûre (10) au moins pendant une partie du mouvement de piqûre.

17. Système de piqûre selon l'une des revendications 13 à 16, **caractérisé en ce que**
il inclut une bande d'éléments aiguilles (331) comprenant une pluralité d'éléments aiguilles (6), fixés et scellés sur une feuille de support (328) de façon à ce que leurs pointes d'aiguille (13) soient protégées de manière hygiénique,
il présente un dispositif de transport, au moyen duquel la bande d'éléments aiguilles (331) est transportée dans l'appareil de piqûre pas à pas de façon à ce que chaque fois un élément aiguille (6) se trouve dans un poste de piqûre (332) près d'un élément de base (333) de l'élément de référence,
il présente un dispositif de préparation de piqûre (335), au moyen duquel l'élément aiguille (6) se trouvant dans le poste de piqûre (332) est amené dans une position de piqûre dans laquelle sa pointe (13) est dégagée et orientée dans le sens de piqûre, tandis que la feuille de support recouvre l'élément de base (333) de l'élément de référence dans le voisinage de la pointe d'aiguille (13) et forme la surface de contact avec la peau (11),
l'élément de base (333) de l'élément de référence, pendant le mouvement de piqûre, étant déplacé conjointement avec la feuille de support (328) formant la surface de contact avec la peau (11) et constituant l'élément de référence profondeur de piqûre (10).

18. Système de piqûre selon la revendication 17, **caractérisé en ce que** la feuille de support a une épaisseur maximale de 100 µm, de préférence de 50 µm et mieux encore de 30 µm.

19. Système de piqûre selon la revendication 17 ou 18, **caractérisé en ce que** éléments aiguilles sont scellés au moyen d'une feuille de recouvrement (329) dont l'épaisseur maximale est de 50 µm, de préférence de 25 µm et mieux encore de 15 µm.

20. Système de piqûre selon l'une des revendications 17 à 19, **caractérisé en ce que** l'élément de référence de profondeur de piqûre (10) sert de support à l'élément aiguille (6) et présente des moyens de fixation au moyen desquels l'élément aiguille (6) peut être fixé dans différentes positions longitudinales par rapport à l'élément de référence de profondeur de piqûre (10) pour régler la profondeur de piqûre.

21. Ensemble de piqûre jetable (1) destiné à être utilisé avec un appareil de piqûre (2) dans lequel chaque fois un ensemble de piqure jetable (1) peut être couplé de manière interchangeable à un entraînement de piqûre (4) prévu dans l'appareil de piqûre (2), comprenant
- un élément aiguille (6) pour piquer la peau et
- un élément de référence de profondeur de piqûre (10) grâce auquel une valeur prédéterminée de la profondeur de piqûre est garantie,
a) l'élément aiguille (6) et l'élément de référence de profondeur de piqûre (10) étant des éléments séparés de l'ensemble de piqûre (1), déplaçables l'un par rapport à l'autre dans le sens de piqûre,
b) l'élément aiguille (6) et l'élément de référence de profondeur de piqûre (10) pouvant être couplés à l'entraînement de l'appareil de piqûre par un mécanisme de couplage (3), de telle sorte que l'élément de référence de profondeur de piqûre est déplacé au moins pendant une partie de la phase d'avancement du mouvement de piqûre conjointement avec l'élément aiguille (6) et a au moins au point d'inversion du mouvement de piqûre une position longitudinale définie dans le sens de piqûre par rapport à l'élément aiguille (6),
c) la position longitudinale dans le sens de piqûre (24) de l'élément de référence de profondeur de piqûre (10) par rapport à l'élément aiguille (6) et donc la distance entre la surface de contact avec la peau (11) et la pointe d'aiguille (13) étant modifiable pour régler la profondeur de piqûre prédéterminée, ladite profondeur de piqûre étant réglable en modifiant la distance (d) existant entre la surface de contact avec la peau (11) de l'élément de référence de profondeur de piqûre (10) et la pointe (13) de l'élément aiguille (6) lorsque le point d'inversion est atteint, et
d) l'élément aiguille (6) présentant une butée (6b) qui coopère avec une butée correspondante (16b) d'un élément de positionnement de l'élément aiguille (16a) du mécanisme de couplage (3), de telle sorte que la position longitudinale de l'élément aiguille (6) est déterminée au moins au point d'inversion du mouvement de piqûre par le contact de ces butées (16b, 6b),
e) l'élément de référence de profondeur de piqûre (10) présentent une butée (10b) qui coopère avec une butée correspondante (17b) d'un élément de positionnement de l'élément de référence (17a) du mécanisme de couplage (3), de telle sorte que la position longitudinale de l'élément de référence de profondeur de piqûre (10) est déterminée au moins au point d'inversion du mouvement de piqûre par le contact de ces butées (17b, 10b).

22. Ensemble de piqûre jetable selon la revendication 21, **caractérisé en ce que** l'élément aiguille (6) présente une structure de couplage qui coopère avec un profil de couplage correspondant de l'élément de positionnement de l'élément aiguille (16a) pour coupler l'élément aiguille (6) à l'élément de positionnement de l'élément aiguille (16a).

23. Ensemble de piqûre jetable selon la revendication 21, **caractérisé en ce que** l'élément de référence de profondeur de piqûre (10) présente également une structure de couplage qui coopère avec un profil de couplage correspondant de l'élément de positionnement de l'élément de référence pour coupler l'élément de référence à l'élément de positionnement de l'élément de référence.
